# EUROPEAN PATENT SPECIFICATION

(11) **EP 1 457 229 B1**
(45) Date of publication and mention of the grant of the patent: **17.12.2008**
(21) Application number: 03251576.9
(22) Date of filing: 14.03.2003
(51) Int. Cl.: A61M 25/06, A61M 5/32

(54) **Intravenous catheter inserting device**
Vorrichtung zum Einführen eines intravenösen Katheters
DISPOSITIF DINSERTION D'UN CATHETER INTRAVEINEUX

(43) Date of publication of application: 15.09.2004
(73) Proprietor: Shue, Ming-Jeng, Hsi Dist, Taichung City (TW); Huang, Deborah, Taichung City (TW)
(72) Inventor: Shue, Ming-Jeng, Taichung City (TW); Huang, Deborah, Chung District, Taichung City (TW); Shue, Philip, Chung District, Taichung City (TW)
(74) Representative: Eddowes, Simon

(56) References cited:
- FR-A- 2 675 999
- US-A- 5 376 071
- US-A- 5 407 431
- US-A- 6 077 245
- US-B1- 6 171 281

## Description

This invention relates to an intravenous catheter inserting device, more particularly to an intravenous catheter inserting device which enables a needle cannula to be retracted within a plunger cavity having a reduced pressure therein.

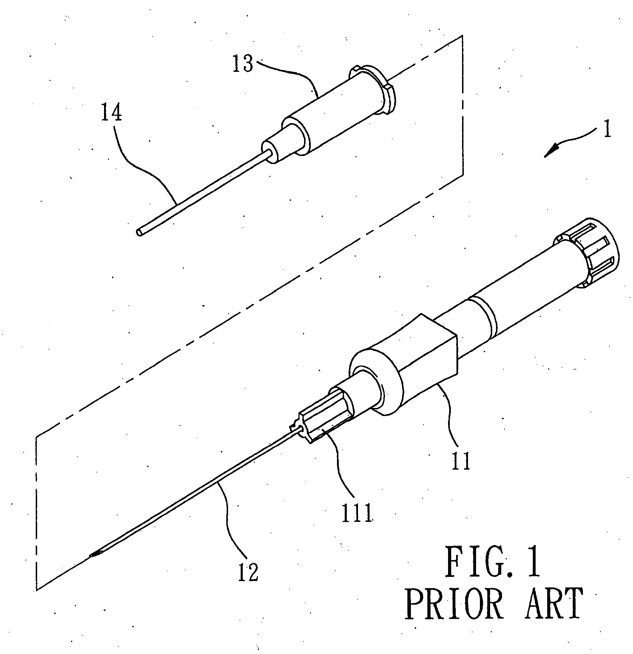

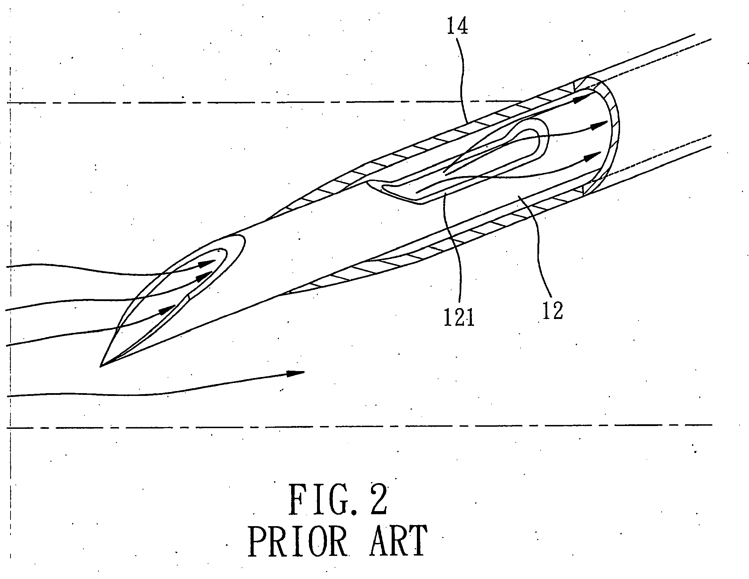

Intravenous catheter inserting devices are generally used to administer medication fluid into or draw blood from a patient's vein. Referring to Figs. 1 and 2, a conventional intravenous catheter inserting device 1 is shown to include a tubular needle seat 11 with a hub end 111, a needle cannula 12 secured to the hub end 111, a catheter hub 13 sleeved on the needle seat 11, and a flexible tubular catheter 14 secured to the catheter hub 13. In use, the catheter 14 and the needle cannula 12 are inserted into the patient' s vein by a health care worker by piercing the patient's vein with a sharp tip of the needle cannula 12 which projects outwardly of the catheter 14.. The health care worker then withdraws the needle cannula 12 from the catheter 14 with one hand and, at the same time, applies pressure to the patient' s skin with the other hand, thereby leaving the catheter 14 in the patient's vein. Subsequently, a transfusion member (not shown) with medication fluid or an empty barrel is connected to the catheter hub 13 for administering the medication fluid into the patient's vein or for drawing blood. At this time, as the health care worker must place the used needle cannula 12 and the needle seat 11 on a tray (not shown) nearby, the exposed sharp tip of the used needle cannula 12 may create a danger of an accidental needle stick. Moreover, blood contamination may occur during connection of the catheter hub 13 to the transfusion member or the empty barrel.

Referring to Fig. 2, during the insertion procedure, the now of blood into the catheter 14 through a notch 121 in the needle cannula 12 can be observed through the translucent catheter 14. However, due to the presence of the notch 121, the needle cannular 12 lends to be bent during passage of the catheter 14 into the patient's vein. In addition, the blood in the catheter 14 is not visible when the portion of the needle cannula 12 with the notch 121 is inserted into the patient's vein.

US-A-5,376,071 (Henderson) describes an approved assembly for insertion of an intravenous catheter and a method of insertion which minimizes or eliminates the potential for exposure to blood and other bodily fluids during the use and insertion of an intravenous catheter. An intravenous catheter hub has a side opening directly connected to a port of a multi-port stopcock. A modified syringe and hollow-bore needle are attached directly to the base of the catheter and when properly inserted, the needle extend slightly beyond the tip of the catheter. After the needle is inserted into the skin, the syringe is used to generate slight negative pressure so that blood immediately flows into the syringe as soon as a vein is punctured by the needle.

FR-A-2,675,999 (Ferras) describes a syringe having a needle cannula which can he retractably received in the open forward end of the syringe barrel. The syringe plunger has a cavity extending in the longitudinal direction and containing fluid at a reduced pressure and which is coupled with the needle cannula within the passage of the barrel such that the needle cannula can be retracted into the cavity once the needle cannula is subject to a suction force that arises a result of a pressure difference between the ambient air and the reduced pressure.

The object of the present invention is to provide an intravenous catheter inserting device which can prevent accidental, inadvertent contact with a needle cannula after use, and which can prevent contamination of that was drawn with the use of the same.

According to this invention, the intravenous catheter inserting device includes a catheter hub, a tubular catheter, a barrel, a needle cannula, a tubular needle scat, and a plunger.

The catheter hub includes a surrounding tip wall which surrounds a first axis and which confines a through hole, a sleeve wall which has an inner sleeve wall surface that confines an insert hole larger than the through hole, and an intermediate tubular wall which is interposed between the surrounding tip wall and the sleeve wall, which confines a duct communicating the insert hole with the through hole, and which has a communicating port to communicate the duct with ambient air.

The tubular catheter includes a proximate segment which is disposed in the through hole and which extends along the first axis to communicate with the duct, and a distal segment which extends from the proximate segment along the first axis to project outwardly of the surrounding tip wall.

The barrel has outer and inner surrounding barrel wall surfaces opposite to each other and surrounding a second axis. The inner surrounding barrel wall surface confines a passage which has opposite open forward and rearward ends, and includes a larger-diameter segment and a smaller-diameter segment that confine rear and front passageways, respectively, and that are disposed proximate to the open rearward and forward ends, respectively, to form a surrounding shoulder portion. The outer surrounding barrel wall surface has a front surrounding region which is secured relative to the inner sleeve wall surface, thereby resulting in coincidence of the first axis with the second axis.

The needle cannula includes opposite tip and fixed ends and a middle segment interposed therebetween.

The tubular needle seat is received in the passage, and extends along the second axis. The tubular needle seat includes a hub end which is disposed to secure the fixed end, a surrounding engaged wall which extends from the hub end along the second axis and which confines an axial path to accommodate or communicate with the fixed end, and an anchoring segment which confines an axial through hole to communicate with the passage and which extends outwardly of the front passageway from the surrounding engaged wall. The surrounding engaged wall has an outer engaged wall surface which engages the smaller-diameter segment and which is rotatable relative to the smaller-diameter segment about the second axis between interengaged and released positions, where the surrounding engaged wall is respectively unmovable and movable relative to the smaller-diameter segment along the second axis, respectively. The anchoring segment terminates at an anchoring end which is rotated with the surrounding engaged wall. As such, when the surrounding engaged wall is turned in a clockwise direction from the interengaged position to the released position, the anchoring end can move from a hook-up position that is closer to the surrounding shoulder portion, to a depressed position that is remote from the surrounding shoulder portion.

The plunger is received in the passage such that in a use position, the plunger is movable along the larger-diameter segment, and such that in a disposal position, the plunger is unmovable along but is rotatable relative to the larger-diameter segment. The plunger includes a plunger body and a seal member.

The plunger body includes a top end wall disposed to confront the surrounding shoulder portion, and a bottom end wall extending outwardly of the open rearward end to permit movement and rotation of the plunger. The top end wall has an inner peripheral edge portion which surrounds the second axis, and which defines a cavity therein. The cavity extends along the second axis and towards the bottom end wall, and contains fluid at a reduced pressure.

The seal member includes an anchored portion and a sealing portion. The anchored portion is disposed to be engageable with the anchoring end such that when the plunger is in the disposal position, and when the surrounding engaged wall is in the interengaged position, rotation of the plunger body in a clockwise direction relative to the larger-diameter segment brings the surrounding engaged wall to turn from the interengaged position to the released position, thereby moving the anchoring end from the hook-up position to the depressed position. The sealing portion is sealingly attached to the inner peripheral edge portion along a sealing line so as to trap the fluid in the cavity. Movement of the anchoring end from the hook-up position to the depressed position results in depression of the anchored portion so as to rip the sealing line, thereby releasing the seal member from the plunger body. Hence, the tubular needle seat together with the needle cannula can be pulled by the seal member which is suctioned into the cavity due to a pressure difference between the reduced pressure and the ambient air that is introduced through the communicating port, thereby permitting retraction of the needle cannula from the tubular catheter into the cavity.

Other features and advantages of the present invention will become apparent in the following detailed description of the preferred embodiments of the invention, with reference to the accompanying drawings, in which:
Fig. 1 is a perspective view of a conventional intravenous catheter inserting device;
Fig. 2 is a partly sectional schematic view illustrating a needle cannula sleeved over by a catheter of the conventional intravenous catheter inserting device;
Fig. 3 is an exploded sectional view of the first preferred embodiment of an intravenous catheter inserting device according to this invention;
Fig. 4 is a sectional view of the first preferred embodiment in a state of use;
Fig. 5 is a sectional view of the first preferred embodiment in a state where a seal member is in contact with a tubular needle seat;
Fig. 6 is a sectional view of the first preferred embodiment in a state where the seal member engages the tubular needle seat;
Fig. 7 is a sectional view of the first preferred embodiment in a state where the seal member is disengaged from a plunger body;
Fig. 8 is a sectional view of the first preferred embodiment in a state where the tubular needle seat and a needle cannula are retracted into the plunger body;
Fig. 9 is a fragmentary sectional view of the first preferred embodiment in another state of use;
Fig. 10 is a fragmentary sectional view of the second preferred embodiment of an intravenous catheter inserting device according to this invention;
Fig. 11 is a sectional view of the third preferred embodiment of an intravenous catheter inserting device according to this invention;
Fig. 12 is a perspective view of the fourth preferred embodiment of an intravenous catheter inserting device according to this invention; and
Fig. 13 is a sectional view of the fourth preferred embodiment of the intravenous catheter inserting device.

Before the present invention is described in greater detail, it should be noted that same reference numerals have been used to denote like elements throughout the specification.

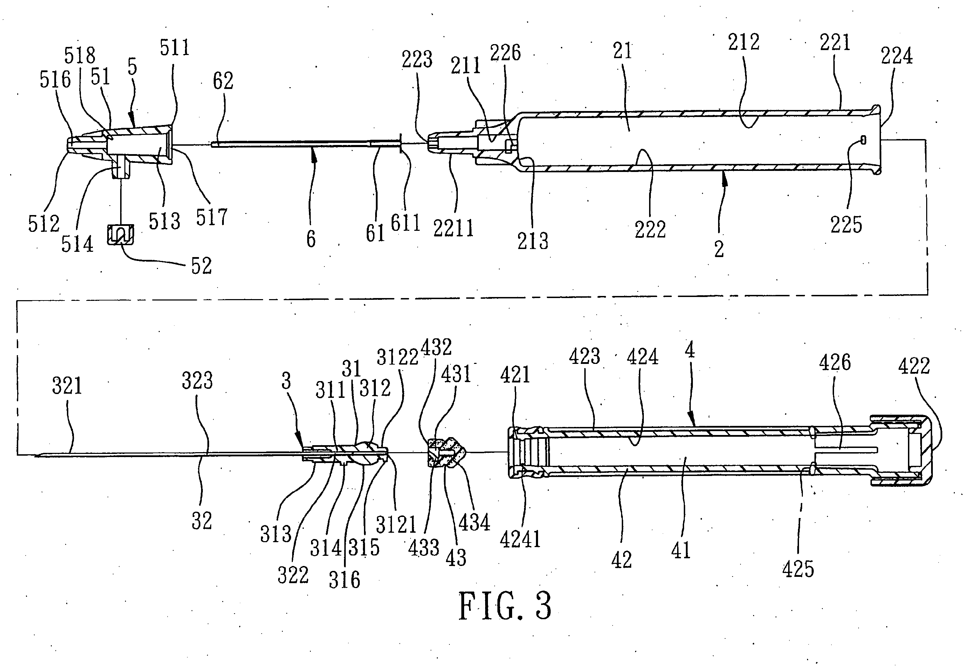

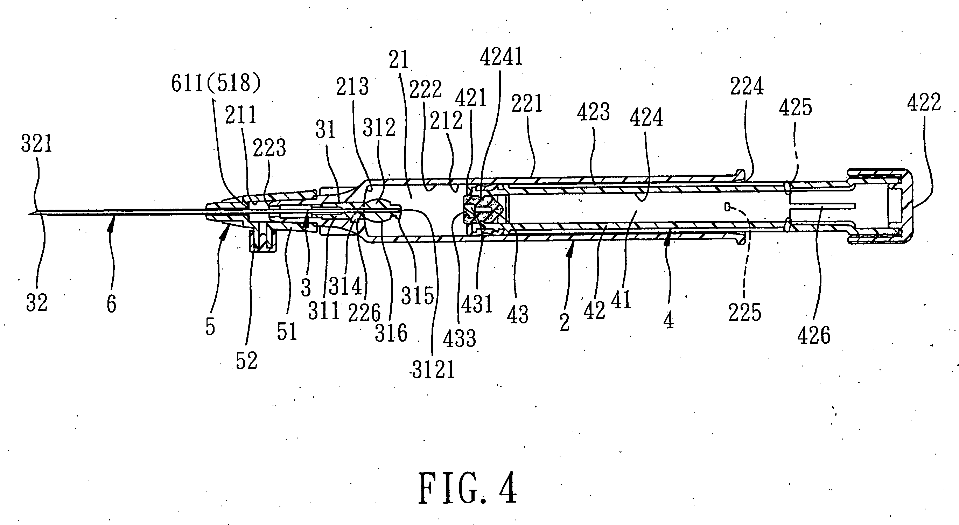

Referring to Figs. 3 and 4, the first preferred embodiment of an intravenous catheter inserting device according to the present invention is shown to comprise a catheter hub 5, a tubular catheter 6, a barrel 2, a needle assembly 3, and a plunger 4.

The catheter hub 5 includes a surrounding tip wall 512, a sleeve wall 511 which is opposite to the surrounding tip wall 512 along a first axis, and an intermediate tubular wall 51 which is interposed between the surrounding tip wall 512 and the sleeve wall 511.

The surrounding tip wall 512 surrounds the first axis, and confines a through hole 516 which extends along the first axis. The sleeve wall 511 has an inner sleeve wall surface which surrounds the first axis and which confines an insert hole 517 larger than the through hole 516. The intermediate tubular wall 51 has an inner tubular wall surface that confines a duct 513 which communicates the insert hole 517 with the through hole 516, and an outer tubular wall surface opposite to the inner tubular wall surface in radial directions. In addition, the intermediate tubular wall 51 has a communicating port 514 which extends radially through the inner and outer tubular wall surfaces to communicate the duct 513 with ambient air. A plug 52 is disposed to close the communicating port 514, as shown in Fig. 4. The inner tubular wall surface of the intermediate tubular wall 51 forms a retaining shoulder 518 with the surrounding tip wall 512. The retaining shoulder 518 confronts the duct 513 along the first axis.

The tubular catheter 6 includes a proximate segment 61 which is disposed in the through hole 516 and which extends along the first axis to communicate with the duct 517 and to project outwardly of the through hole 516, and a distal segment 62 which extends from the proximate segment 61 along the first axis to project outwardly of the surrounding tip wall 512. The proximate segment 61 terminates at a flange portion 611 which abuts against and which is retained at the retaining shoulder 518.

The barrel 2 has outer and inner surrounding barrel wall surfaces 221,222 opposite to each other and surrounding a second axis. The inner surrounding barrel wall surface 222 confines a passage 21 which has open forward and rearward ends 223,224 that are disposed opposite to each other in a longitudinal direction parallel to the second axis, and includes a larger-diameter segment 212 and a smaller-diameter segment 211 which confine rear and front passageways, respectively, and which are disposed proximate to the open rearward and forward ends 224,223, respectively, to form a surrounding shoulder portion 213 between the larger-diameter segment 212 and the smaller-diameter segment 211. The outer surrounding barrel wall surface 221 has a front surrounding region 2211 which is proximate to the open forward end 223, and which is insertable into the insert hole 517 so as to abut against the inner sleeve wall surface of the sleeve wall 511, thereby resulting in coincidence of the first axis with the second axis. A first female screw thread segment 225 includes a plurality of angularly displaced ribs, and is disposed on the larger-diameter segment 212 adjacent to the open rearward end 224. A second female screw thread segment 226 in the form of a spiral groove is disposed on the smaller-diameter segment 211 adjacent to the surrounding shoulder portion 213.

The needle assembly 3 includes a needle cannula 32 and a tubular needle seat 31.

The needle cannula 32 includes tip and fixed ends 321,322 opposite to each other, and a middle segment 323 interposed between the tip and fixed ends 321,322.

The tubular needle seat 31 is made of a light transmissible material, is received in the passage 21, and extends along the second axis. The tubular needle seat 31 includes a hub end 313 which is disposed to secure the fixed end 322 of the needle cannula 32, and a surrounding engaged wall 311 which extends from the hub end 313 along the second axis, and which confines an axial path to accommodate or communicate with the fixed end 322 of the needle cannula 32. The surrounding engaged wall 311 has an outer engaged wall surface which is provided with a (second) male screw thread segment 314 that includes a plurality of angularly displaced ribs and that surrounds the second axis to threadedly engage the second female screw thread segment 226 in an interengaged position, whereby the surrounding engaged wall 311 of the tubular needle seat 31 is unmovable relative to the smaller-diameter segment 211 along the second axis.

The tubular needle seat 31 further includes an anchoring segment 312 which confines an axial through hole 3121 to communicate with the passage 21, and which extends outwardly of the front passageway of the smaller-diameter segment 211 from the surrounding engaged wall 311 to terminate at an anchoring end 3122. A (third) male screw thread segment 315 includes a plurality of ribs and is disposed on the anchoring end 3122.

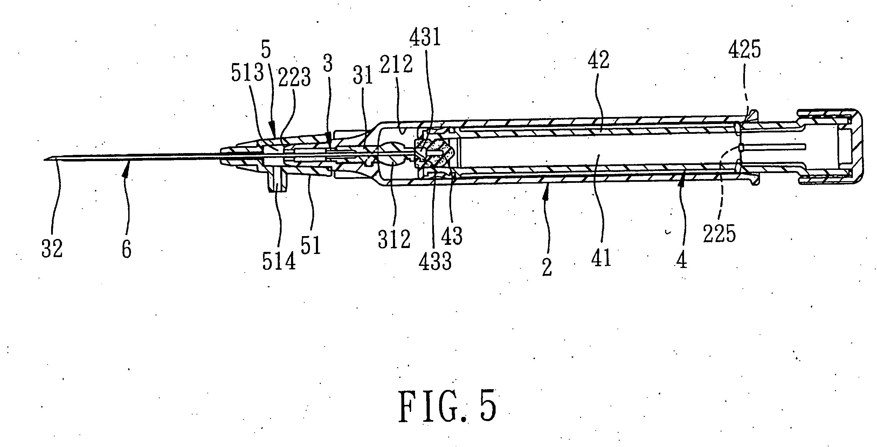

The plunger 4 is received in the passage 21 such that in a use position, as shown in Fig. 4, the plunger 4 is movable along the larger-diameter segment 212 in the longitudinal direction, and such that in a disposal position, as shown in Fig. 5, the plunger 4 is unmovable along, but is rotatable relative to the larger-diameter segment 212. The plunger 4 includes a plunger body 42 and a seal member 43.

The plunger body 42 includes a top end wall 421 which is disposed to confront the surrounding shoulder portion 213, and a bottom end wall 422 opposite to the top end wall 421 in the longitudinal direction. The bottom end wall 422 extends outwardly of the open rearward end 224 to permit movement and rotation of the plunger 4. The top end wall 421 has an inner peripheral edge portion 424 which surrounds the second axis, and which defines a cavity 41 therein. The cavity 41 extends along the second axis and towards the bottom end wall 422, and contains fluid at a reduced pressure. In addition, the plunger body 42 has an outer plunger wall surface 423 which surrounds the second axis. A first male screw thread segment 425 includes a plurality of ribs, and is disposed on the outer plunger wall surface 423. A catcher portion 426 is disposed on the inner peripheral edge portion 424 proximate to the bottom end wall 422.

The seal member 43 is formed from an elastomeric material, and includes an anchored portion 432 and a sealing portion 434. The anchored portion 432 has an inner surrounding wall which extends along the second axis to confine a recess 431 that is configured to accommodate and to be engageable with the anchoring end 3122 of the anchoring segment 312. The inner surrounding wall is formed with a third female screw thread segment 433 that is in the form of a spiral groove. Referring to Fig. 4, a surrounding protrusion 4241 is disposed to extend from the inner peripheral edge portion 424 in a radial direction and towards the second axis so as to be in frictional contact with the sealing portion 434, thereby establishing a sealing line to trap the fluid in the cavity 41.

In use, as shown in Fig. 4, a health care worker holds the barrel 2 with one hand to insert the tip end 321 of the needle cannula 32 and the distal segment 62 of the tubular catheter 6 into the patient's vein. The anchoring segment 312 of the tubular needle seat 31 is light transmissible and has a convex surface 316 to provide a magnified view of the blood flowing through the axial through hole 3121. Once the health care worker notes that the blood does not flow into the anchoring segment 312, he/she can pull the plunger 4 slightly rearward to draw the blood until the blood is visible through the anchoring segment 312 so as to confirm correct insertion of the tubular catheter 6 into the patient's vein.

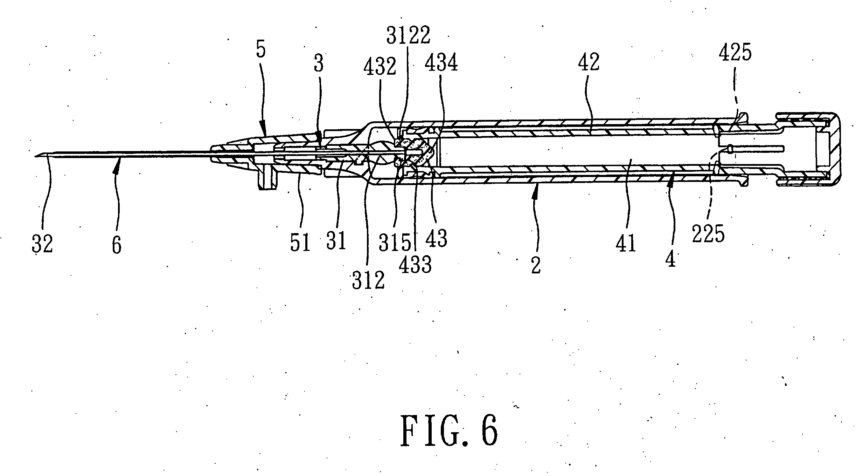

Referring to Figs. 5 and 6, the plug 52 shown in Fig. 4 is removed from the communicating port 514 so as to communicate the duct 513 with the ambient air. The plunger 4 is pressed forwardly to contact the tubular needle seat 31 (see Fig. 5) . By means of the first male and female screw thread segments 425,225, displacement of the plunger 4 along the larger-diameter segment 212 is restrained, whereas rotation of the plunger 4 in a clockwise direction relative to the larger-diameter segment 212 is permitted. The plunger 4 is screwed toward the open forward end 223. By the screw movement of the plunger 4, the seal member 43 is rotated relative to the tubular needle seat 31, and the anchored portion 432 of the seal member 43 is brought to engage the anchoring end 3122 by virtue of the engagement of the third male and female screw thread segments 315,433, as shown in Fig. 6. Thus, the tubular needle seat 31 is rotatable together with the seal member 43.

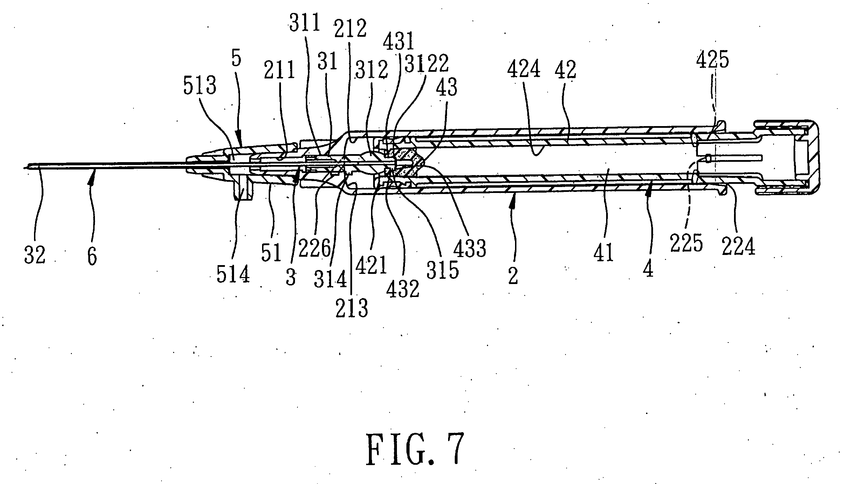

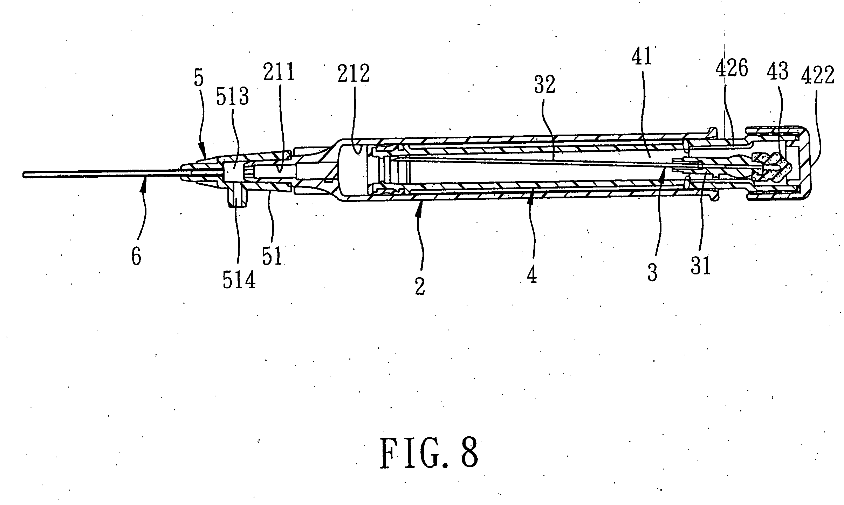

Subsequently, as shown in Fig. 7, when the plunger 4 is further rotated in the clockwise direction relative to the larger-diameter segment 212 such that the tubular needle seat 31 is rotated to turn the second male screw thread segment 314 about the second axis to a released position for removal from the second female screw thread segment 226, the surrounding engaged wall 311 of the tubular needle seat 31 is moved relative to the smaller-diameter segment 211 along the second axis towards the open rearward end 224. The anchoring end 3122 is also rotated to move from a hook-up position that is closer to the surrounding shoulder portion 213 to a depressed position that is remote from the surrounding shoulder portion 213. At the same time, movement of the anchoring end 3122 from the hook-up position to the depressed position will result in depression of the anchored portion 432 so as to rip the sealing line, thereby releasing the seal member 43 from the plunger body 42. As such, the tubular needle seat 31 together with the needle cannula 32 can be pulled by the seal member 43 that is suctioned into the cavity 41 due to the pressure difference between the reduced pressure and the ambient air that is introduced through the communicating port 514, thereby permitting retraction of the needle cannula 32 from the tubular catheter 6 into the cavity 41, as shown in Fig. 8. Furthermore, by means of the catcher portion 426, the tubular needle seat 31 as well as the needle cannula 32 can be prevented from impinging upon the bottom end wall 422.

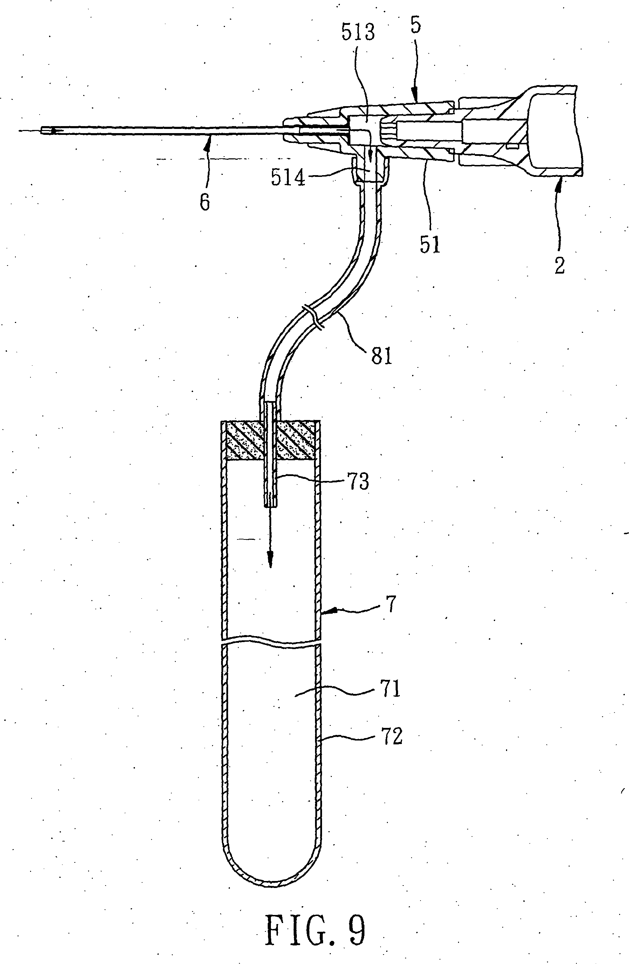

Therefore, in use, the health care worker can close the communicating port 514 with the plug 52, and then pull the plunger body 42, in which the seal member 43 and the needle assembly 3 are left, towards the open rearward end 224 so as to draw a smaller amount of blood into the passage 21 of the barrel 2 through the tubular catheter 6. Referring to Fig. 9, when the health care worker needs to draw a larger amount of blood, a blood collecting member 7 is connected to the catheter hub 5. The blood collecting member 7 includes a surrounding wall 72 which confines a receiving space 71 and which has an introduced port 73 communicating with the receiving space 71. A first connecting flexible tube 81 is disposed to interconnect the introduced port 73 and the communicating port 514. The blood can flow into the blood collecting member 7 through the tubular catheter 6, the duct 513, and the first connecting flexible tube 81 without the need to pull the plunger body 42, thereby preventing the health care worker from coming into contact with the blood.

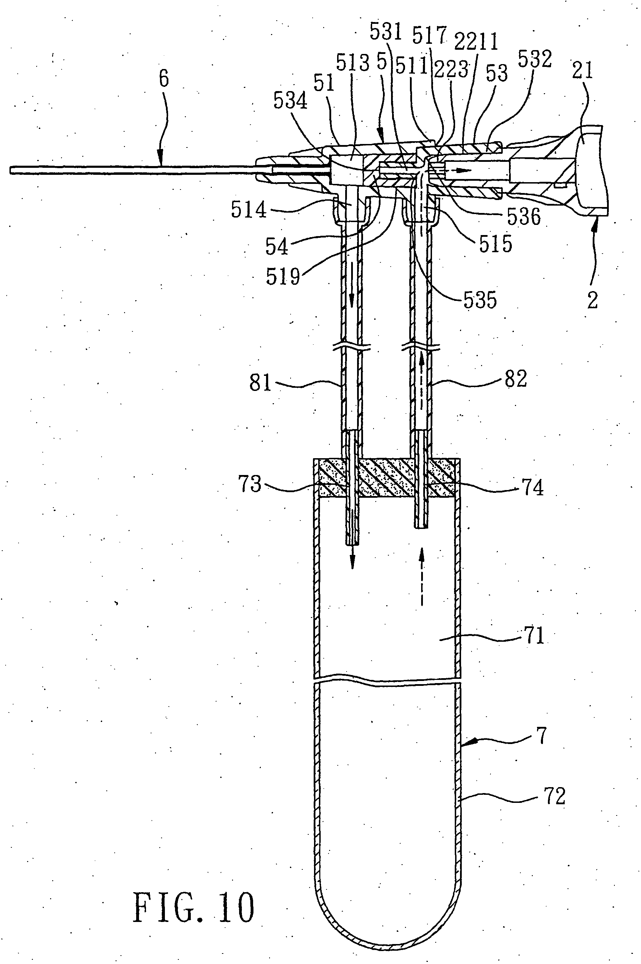

Alternatively, referring to Fig. 10, the second preferred embodiment of the intravenous catheter inserting device according to this invention is shown to be similar to the previous embodiment in construction. In addition to the component parts of the previous embodiment, the inner sleeve wall surface of the sleeve wall 511 of the catheter hub 5 has a reentry port 515 which extends radially through the sleeve wall 511 to communicate with the insert hole 517, and which is opposite to the communicating port 514 relative to a boundary area 519. Moreover, the intravenous catheter inserting device of this embodiment further includes a barrier member 54 and a tubular insert 53. The barrier member 54 is formed from an elastomeric material, such as a rubber. The tubular insert 53 has a front neck end 534 which is connected to the barrier member 54 and which confines a through way 531 along the first axis, and a rear flared end 532 which extends from the front neck end 534 along the first axis, and which has an inner abutted surface 536. The inner abutted surface 536 surrounds the first axis, and confines a fluid path 535 which extends to communicate with the reentry port 515. Furthermore, the blood collecting member 7 has an air outlet 74 spaced apart from the introduced port 73. A second connecting flexible tube 82 is disposed to interconnect the air outlet 74 and the reentry port 515. In assembly, the barrier member 54, on which the front neck end 534 is connected, is engaged with the inner sleeve wall surface of the sleeve wall 511 of the catheter hub 5 at the boundary area 519. The front surrounding region 2211 of the barrel 2 is engaged with the inner abutted surface 536 of the tubular insert 53, thereby aligning the through way 531 with the passage 21 of the barrel 2. The needle cannula 32 (not shown in Fig. 10) extends through the through way 531, a slit in the barrier member 54, the duct 513, and the tubular catheter 6. After the used needle assembly 3 (not shown in Fig. 10) has been retracted into the plunger body (not shown in Fig. 10) in the same manner as described above, the slit in the barrier member 54 is closed by means of its elastomeric material property.

As such, due to the arrangement of the barrier member 54, once it is noted that blood cannot flow into the blood collecting member 7, the health care worker can pull the plunger body 42 rearwardly so as to introduce an air flow from the air outlet 74 through the second connecting flexible tube 82, the reentry port 515, and the fluid path 535 to the passage 21 of the barrel 2, thereby facilitating blood flow into the blood collecting member 7.

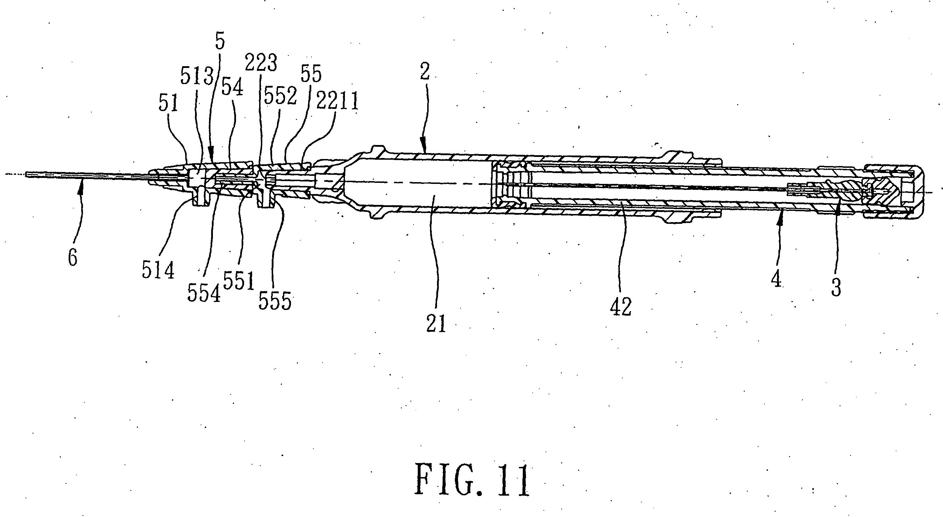

Alternatively, referring to Fig. 11, the third preferred embodiment of the intravenous catheter inserting device according to this invention is shown to be similar to the first preferred embodiment in construction. The duct 513 of the catheter hub 5 is communicated with the passage 21 of the barrel 2 through a tubular insert 55. The tubular insert 55 has a front neck end 554 which is connected to a barrier member 54 and which confines a through way along the axis, and a rear flared end 552 which extends from the front neck end 554 along the first axis. The rear flared end 552 has an inner abutted surface which surrounds the first axis and which confines a fluid path 551, and a reentry port 555 which extends radially therethrough to communicate with the fluid path 551. The front surrounding region 2211 of the barrel 2 engages the inner abutted surface of the tubular insert 55. As such, in the same manner as that of the second preferred embodiment, after the used needle assembly 3 has been retracted into the plunger body 42, the slit in the barrier member 54 is closed by means of its elastomeric material property.

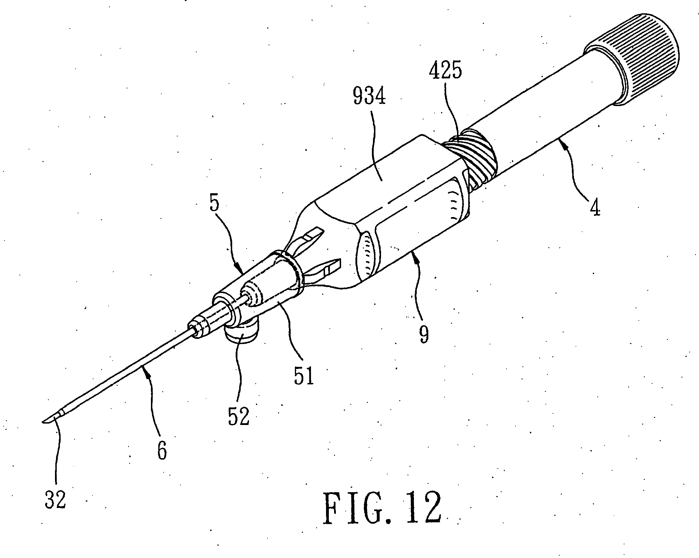

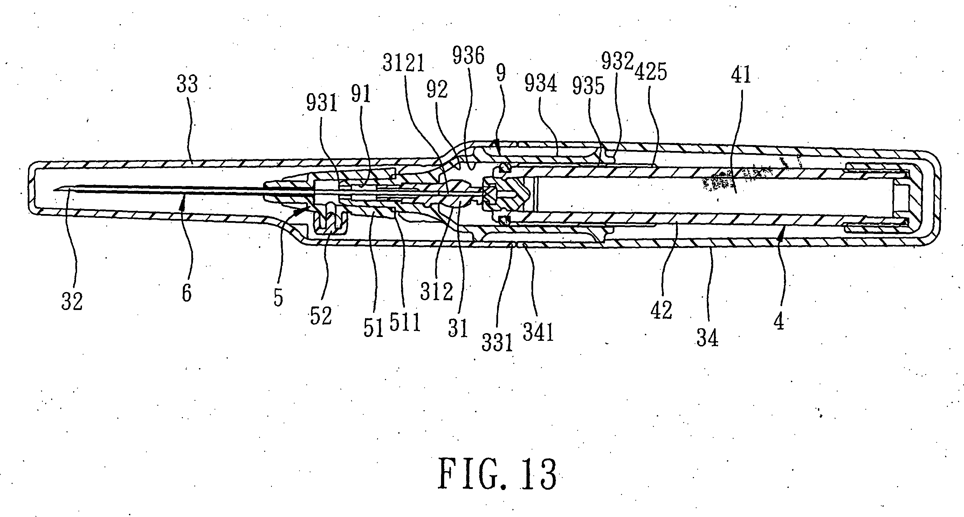

Referring to Figs. 12 and 13, the fourth preferred embodiment of the intravenous catheter inserting device according to this invention is shown to include a barrel 9 which is shorter than that of the previous embodiments. The barrel 9 also has an inner surrounding barrel wall surface 936 which includes smaller-diameter and larger-diameter segments 91,92 and which confines a passage having open forward and rearward ends 931,932. The sleeve wall 511 of the catheter hub 5 is brought to engage the open forward end 931. The larger-diameter segment 92 has a male screw thread segment 935 so as to threadedly engage the female screw thread segment 425 formed on the outer plunger wall surface of the plunger 4. As such, in use, the health care worker can grasp the outer surrounding barrel wall surface 934 and insert the needle cannula 32, as well as the tubular catheter 6, into the patient's vein such that blood can flow into the barrel 9. Then, in the same manner described hereinabove, the needle cannula 32 can be retracted into the cavity 41 of the plunger 4 for safe disposal.

Furthermore, a tip protector 33 is disposed to shield the needle cannula 32 and the catheter hub 5, and has a larger open end 331 for insertion of a front half portion of the barrel 9. A sleeve tube 34 is disposed to shield the plunger 4, and has an open end 341 for insertion of a rear half portion of the barrel 9. Thus, the needle cannula 32 and the plunger 4 can be completely shielded by the tip protector 33 and the sleeve tube 34 so as to prevent the health care worker from coming into contact therewith.

As illustrated, the intravenous catheter inserting device of this invention has the following advantages:
1. After insertion of the needle cannula 32, by rotating the plunger body 42 in the clockwise direction relative to the larger-diameter segment 212, the used needle assembly 3 can be retracted into the cavity 41 of the plunger body 42, thereby facilitating safe disposal of the intravenous catheter inserting device.
2. Since the anchoring segment 312 of the tubular needle seat 31 is light-transmissible and has the convex surface 316, blood flowing through the axial through hole 3121 is visible to the health care worker in a magnified state.
3. After the used needle assembly 3 has been retracted into the cavity 41, the health care worker can directly pull the plunger body 42 for drawing blood into the passage 21 or the blood collecting member 7 without the need to remove the barrel 2 and the catheter hub 5, thereby reducing the possibility of the health care worker coming into contact with blood.

## Claims

1. An intravenous catheter inserting device, **characterized by**:
a catheter hub (5) including
a surrounding tip wall (512) which surrounds a first axis, and which confines a through hole (516) extending along the first axis,
a sleeve wall (511) which is opposite to said surrounding tip wall (512) along the first axis, and which has an inner sleeve wall surface that surrounds the first axis, and that confines an insert hole (517) larger than said through hole (516), and
an intermediate tubular wall (51) which is interposed between said surrounding tip wall (512) and said sleeve wall (511), which confines a duct (513) communicating said insert hole (517) with said through hole (516), and which has a communicating port (514) extending therethrough to communicate said duct (513) with ambient air;
a tubular catheter (6) including a proximate segment (61) which is disposed in said through hole (516) and which extends along the first axis to communicate with said duct (513), and a distal segment (62) which extends from said proximate segment (61) along the first axis to project outwardly of said surrounding tip wall (512);
a barrel (2,9) having outer and inner surrounding barrel wall surfaces (221,222) opposite to each other and surrounding a second axis, said inner surrounding barrel wall surface (222) confining a passage (21) having open forward and rearward ends (223,224,931,932) that are disposed opposite to each other in a longitudinal direction parallel to the second axis, said inner surrounding barrel wall surface (222) including a larger-diameter segment (212, 92) and a smaller-diameter segment (211,91) that confine rear and front passageways, respectively, and that are disposed proximate to said open rearward and forward ends (224, 223, 932, 931), respectively, to form a surrounding shoulder portion (213) between said larger-diameter segment (212,92) and said smaller-diameter segment (211,91), said outer surrounding barrel wall surface (221) having a front surrounding region (2211) which is proximate to said open forward end (223, 931), and which is secured relative to said inner sleeve wall surface, thereby resulting in coincidence of the first axis with the second axis;
a needle cannula (32) including tip and fixed ends (321,322) opposite to each other, and a middle segment (323) interposed between said tip and fixed ends (321,322);
a tubular needle seat (31) received in said passage (21) and extending along the second axis, said tubular needle seat (31) including
a hub end (313) disposed to secure said fixed end (322),
a surrounding engaged wall (311) which extends from said hub end (313) along the second axis, and which confines an axial path to accommodate or communicate with said fixed end (322), said surrounding engaged wall (311) having an outer engaged wall surface which surrounds the second axis, and which engages and which is rotatable relative to said smaller-diameter segment (211,91) about the second axis between interengaged and released positions, where said surrounding engaged wall (311) is unmovable and movable relative to said smaller-diameter segment (211, 91) along the second axis, respectively, and
an anchoring segment (312) which confines an axial through hole (3121) to communicate with said passage (21), and which extends outwardly of said front passageway from said surrounding engaged wall (311), said anchoring segment (312) terminating at an anchoring end (3122) which is rotated with said surrounding engaged wall (311) to move from a hook-up position that is closer to said surrounding shoulder portion (213), to a depressed position that is remote from said surrounding shoulder portion (213) when said surrounding engaged wall (311) is turned in a clockwise direction from the interengaged position to the released position; and
a plunger (4) received in said passage (21) such that in a use position, said plunger (4) is movable along said larger-diameter segment (212,92) in the longitudinal direction, and such that in a disposal position, said plunger (4) is unmovable along but is rotatable relative to the larger-diameter segment (212,92), said plunger (4) including
a plunger body (42) which includes a top end wall (421) disposed to confront said surrounding shoulder portion (213), and a bottom end wall (422) opposite to said top end wall (421) in the longitudinal direction, said bottom end wall (422) extending outwardly of said open rearward end (224,932) to permit movement and rotation of said plunger (4), said top end wall (421) having an inner peripheral edge portion (424) which surrounds the second axis, and which defines a cavity (41) therein, said cavity (41) extending along the second axis and towards said bottom end wall (422) and containing fluid at reduced pressure, and
a seal member (43) including
an anchored portion (432) disposed to be engageable with said anchoring end (3122) such that when said plunger (4) is in the disposal position and when said surrounding engaged wall (311) is in the interengaged position, rotation of said plunger body (42) in the clockwise direction relative to said larger-diameter segment (212,92) brings said surrounding engaged wall (311) to turn from the interengaged position to the released position, thereby moving said anchoring end (3122) from the hook-up position to the depressed position, and
a sealing portion (434) which is sealingly attached to said inner peripheral edge portion (424) along a sealing line so as to trap said fluid in said cavity (41), the sealing line being configured such that movement of said anchoring end (3122) from the hook-up position to the depressed position results in depression of said anchored portion (432) so as to rip the sealing line, thereby releasing said seal member (43) from said plunger body (42), so that said tubular needle seat (31) together with said needle cannula (32) will be pulled by said seal member (43) which is suctioned into said cavity (41) due to a pressure difference-between the reduced pressure and the ambient air that is introduced through said communicating port (514), thereby permitting retraction of said needle cannula (32) from said tubular catheter (6) into said cavity (41).

2. The intravenous catheter inserting device according to Claim 1, **characterized in that** said front surrounding region (2211) of said outer surrounding barrel wall surface (221) of said barrel (2) is inserted into said insert hole (517) so as to abut against said inner sleeve wall surface of said sleeve wall (511) of said catheter hub (5).

3. The intravenous catheter inserting device according to Claim 2, **characterized in that** said plunger body (42) has an outer plunger wall surface (423) which surrounds the second axis, said intravenous catheter inserting device further comprising first male and female screw thread segments (425,225) respectively disposed on said outer plunger wall surface (423) and said larger-diameter segment (212,92) to restrain said plunger body (42) from displacing along said larger-diameter segment (212, 92) while permitting rotation of said plunger body (42) in a clockwise direction relative to said larger-diameter segment (212,92) in the disposal position.

4. The intravenous catheter inserting device according to Claim 3, further **characterized by** second male and female screw thread segments (314,226) respectively disposed on said outer engaged wall surface and said smaller-diameter segment (211,91) to permit rotation of said outer engaged wall surface relative to said smaller-diameter segment (211,91).

5. The intravenous catheter inserting device according to Claim 4, **characterized in that** said anchoring segment (312) is made of a material that is light transmissible so as to permit viewing of liquid, flowing through said axial through hole (3121).

6. The intravenous catheter inserting device according to Claim 5, **characterized in that** said anchoring segment (312) is configured in such a manner so as to provide a magnified view of blood in said axial through hole (3121).

7. The intravenous catheter inserting device according to Claim 6, **characterized in that** said seal member (43) is formed from an elastomeric material.

8. The intravenous catheter inserting device according to Claim 7, **characterized in that** said anchored portion (432) has an inner surrounding wall which extends along the second axis to confine a recess (431) that is configured to accommodate said anchoring end (3122), said intravenous catheter inserting device further comprising third male and female screw thread segments (315,433) disposed respectively on said anchoring end (3122) and said inner surrounding wall so as to permit said anchored portion (432) to be brought to engage with said anchoring end (3122) by virtue of rotation of said third male and female screw thread segments (315,433) relative to each other.

9. The intravenous catheter inserting device according to Claim 8, further **characterized by** a surrounding protrusion (4241) disposed to extend from said inner peripheral edge portion (424) in radial directions and towards the second axis so as to be in frictional contact with said sealing portion (434), thereby establishing the sealing line.

10. The intravenous catheter inserting device according to Claim 9, further **characterized by** a catcher portion (426) disposed on said inner peripheral edge portion (424) proximate to said bottom end wall (422) and configured to prevent said tubular needle seat (31) and said needle cannula (32) from impinging upon said bottom end wall (422).

11. The intravenous catheter inserting device according to Claim 1, **characterized in that** said inner sleeve wall surface of said sleeve wall (511) confines a boundary area (519) which surrounds the first axis, and has a reentry port (515) which extends radially through said sleeve wall (511) to communicate with said insert hole (517) and which is opposite to said communicating port (514) relative to said boundary area (519), said intravenous catheter inserting device further comprising: a barrier member (54) which engages said inner sleeve wall surface at said boundary area (519); and
a tubular insert (53) which has a front neck end (534) that is connected to said barrier member (54) and that confines a through way (531) along the first axis, and a rear flared end (532) that extends from said front neck end (534) along the first axis and that has an inner abutted surface (536), said inner abutted surface (536) surrounding the first axis and confining a fluid path (535) which extends to communicate with said reentry port (515), said inner abutted surface (536) engaging said front surrounding region (2211) of said outer surrounding barrel wall surface (221) of said barrel (2), thereby aligning said through way (531) with said passage (21) of said barrel (2) so as to permit said needle cannula (32) to extend through said through way (531) and outwardly of said tubular catheter (6).

12. An intravenous catheter device for use with a disposable syringe which includes
a barrel (2, 9) having an inner surrounding barrel wall surface (222) which confines a passage (21) that extends in a longitudinal directions and that has open forward and rearward ends (223,224,931,932) opposite to each other in the longitudinal direction, and an outer surrounding barrel wall surface (221) which has a font surrounding region (2211) that is proximate to the open forward end (223, 931),
a needle cannula (32) retractably receive in the open forward end (223,931), and
a plunger (4) which is received in the passage (21) to be movable in the longitudinal direction, which extends outwardly o:f the open rearward end (224, 932) so as to be manually operable, which has a cavity (41) extending in the longitudinal direction and containing fluid at a reduced pressure, and which is coupled with the needle cannula (32) within the passage (21) such that the needle cannula (32) will be retracted into the cavity (41) once the needle cannula (32) is subjected to a suction force that arises as a result of a pressure difference between the ambient air and the reduced pressure, said intravenous catheter device *being **characterized by***:
a catheter hub (5) including
a surrounding tip wall (512) which surrounds an axis, and which confines a through hole (516) extending along the axis,
a sleeve wall (511) which is opposite to said surrounding tip wall (512) along the axis, and which has an inner sleeve wall surface that surrounds the axis, and that confines an insert hole (517) larger than said through hole (516), said inner sleeve wall surface being adapted to be secured relative to the front surrounding region (2211) of the outer surrounding barrel wall surface (221) of the barrel (2) so as to bring the passage (21) into alignment with said through hole (516), and
an intermediate tubular wall (51) which is interposed between said surrounding tip wall (512) and said sleeve wall 1. (511), which confines a duct (513) that communicates said insert hole (517) with said through hole (516), and that is adapted for the needle cannula (32) to pass trough when said inner sleeve wall surface is secured relative to the front surrounding region (2211) of the outer surrounding barrel wall surface (221) of the barrel (2) , and which has a communicating port (514) extending therethrough to communicate said duct (513) with ambient air; and
a tubular catheter (6) including a proximate segment (61) which is disposed in said through hole (516) and which extends along the axis to communicate with said duct (513), and a distal segment (62) which extends from said proximate segment (61) along the axis to project outwardly of said surrounding tip wall (512), and which is adapted to be sleeved on the needle cannula (32) and to permit the needle cannula (32) to extend outwardly thereof, when the needle cannula (32) is brought to pass through said duct (513);
said intermediate tubular wall (51) having an inner tubular wall surface to confine said duct (513), and an outer tubular wall surface opposite to said inner tubular wall surface in radial directions, said inner tubular wall surface forming a retaining shoulder (518) with said surrounding tip wall (512), said retaining shoulder (518) confronting said duct (513) along the axis, said proximate segment (61) extending along the axis to project outwardly of said through hole (516) and terminating at a flange portion (611) which abuts against and which is retained at said retaining shoulder (518);
said communicating port (514) extending radially through said inner and outer tubular wall surfaces;
said inner sleeve wall surface confining a boundary area (519) which surrounds the axis, and having a reentry port (515) which extends radially through said sleeve wall (511) to communicate with staid insert hole (517), and which is opposite to said communicating port (514) relative to said boundary area (519);
said intravenous catheter device further comprising:
a barrier member (54) which engages said inner sleeve wall surface at said boundary area (519); and
a tubular insert (53) which has a front neck end (534) that is connected to said barrier member (54) and that confines a through way (531) along the axis, and a rear flared end (532) that extends from said front neck end (534) along the axis and that has an inner abutted surface (536), said inner abutted surface (536) surrounding the axis and confining a fluid path (535) which extend to communicate with said reentry port (515), said inner abutted surface (536) being adapted to engage the front surrounding region (2211) of the outer surrounding barrel wall surface (221) of the barrel (2), thereby aligning said through way (531) with the passage (21) of the barrel (2) so as to permit the needle cannula (32) to extend through said through way (531) and outwardly of said tubular catheter (6).

13. The intravenous catheter device according to Claim 12, **characterized in that** said barrier member (54) is formed from an elastomeric material.

14. An intravenous catheter device for use with a disposable syringe which includes
a barrel (2, 9) having an inner surrounding barrel wall surface (222) which confines a passage (21) that extends in a longitudinal direction and that has open forward and rearward ends (223, 224, 931, 932) opposite to each other in the longitudinal direction, and an outer surrounding barrel wall surface (221) which has a front surrounding region (2211) that is proximate to the open forward end (223, 931),
a needle cannula (32) retractably received in the open forward end (223,931), and
a plunger (4) which is received in the passage (21) to be movable in the longitudinale direction, which extends outwardly of the open rearward end (224,932) so as top be manually operable, which has a cavity (41) extending in the longitudinal direction and containing fluid at a reduced pressure, and which is coupled with the needle cannula (32) within the passage (21) such that the needle cannula (32) will he retracted into the cavity (41) once the needle cannula (32) is subjected to a section force that arises as a result of a pressure difference between the ambient air and the reduced pressure, said intravenous catheter device *being*
***characterized by**:*
a catheter hub (5) including
a surrounding tip wall (512) which surrounds an axis, and which confines a through halo (516) extending along the axis,
a sleeve wall (511) which is opposite to said surrounding tip wall (512) along the axis, and which has an inner sleeve wall surface that surrounds the axis, and that confines an insert hole (517) larger than said through hole (516), said inner sleeve wall surface being adapted to be secured relative to the front surrounding region (2211) of the outer surrounding barrel wall surface (221) of the barrel (2) so as to bring the passage (21) into alignment with said through hole (516), and
an intermediate tubular wall (51) which is interposed between said surrounding tip wall (512) and said sleeve wall (511), which confines a duct (513) that communicates said insert hole (517) with said through hole (516), and that is adapted for the needle cannula (32) to pass through when said inner sleeve wall surface is secured relative to the front surrounding region (2211) or the outer surrounding barrel wall surface (221) of the barrel (2), and which has a communicating port (514) extending therethrough to communicate said duct (513) with ambient air; and
a tubular catheter (6) including a proximate segment (61) which is disposed in said through hole (516) and which extends along the axis to communicate with said duct (513), and a distal segment (62) which extends from said proximate segment (61) along the axis to project outwardly of said surrounding tip wall (512), and which is adapted to be sleeved on the needle cannula (32) and to permit the needle cannula (32) to extend outwardly thereof, when the needle cannula (32) is brought to pass through said duct (513):
said intermediate tubular wall (51) having an inner tubular wall surface to confine said duct (513), and an outer tubular wall surface opposite to said inner tubular wall surface in radial directions, said inner tubular wall surface forming a retraining shoulder (518) with said surrounding tip wall (512), said retaining shoulder (518) confronting said duct (513) along the axis, said proximate segment (61) extending along the axis to project outwardly of said through hole (516) and terminating at a flange portion (611) which abuts against and which is retained at said retaining shoulder (518) ;
said communicating port (514) extending radically through said inner and outer tubular wall surfaces;
said inner sleeve wall surface confining a boundary area which surrounds the axis;
said intravenous catheter device further comprising:
a barrier member (54) which engages said inner sleeve wall surface at said boundary area; and
a tubular insert (55) which has a front neck end (554) that is connected to said barrier member (54) and that confines a through way along the axis, and a rear flared end (552) that extends from said front neck end (554) along the axis, said rear flared end (552) having an inner abutted surface which surrounds the axis, and which confines a fluid path (551), and a reentry port (555) which extends radially to communicate with said fluid path (551) and which is opposite to said communicating port (514) relative to said boundary area, said inner abutted surface of said rear flared end (552) being adapted to engage the front surrounding region (2211) of the outer surrounding barrel wall surface (221) of the barren (2), thereby aligning said through way with the passage (21) of the barrel (2) so as to permit the needle cannula (32) to extend through said through way and outwardly of said tubular catheter (6).

## Patentansprüche

1. Gerät zum Insertieren eines intravenösen Katheters, **gekennzeichnet durch**:
eine Katheternabe (5), die Folgendes umschließt:
eine umgebende Spitzenwand (512), die eine erste Achse umgibt und ein hindurchgehendes Loch (516) umschließt, das sich der ersten Achse entlang erstreckt.
eine Buchsenwand (511), die der umgebenden Spitzenwand (512) der ersten Achse entlang gegenüberliegt und die eine innere Buchsenwandfläche aufweist, die die erste Achse umgibt und die ein Insertionsloch (517) umschließt, das größer als das hindurchgehende Loch (516) ist und
eine Zwischenröhrenwand (51), die zwischen die umgebende Spitzenwand (512) und die Buchsenwand (511) eingeschoben ist, die eine Durchführung (513) umschließt, die das Insertionsloch (517) mit dem hindurchgehenden Loch (516) in Kommunikation bringt und die eine Kommunikationsöffnung (514) aufweist, die sich dort hindurch erstreckt, um die Durchführung (513) mit der Umgebungsluft in Kommunikation zu bringen;
einen Röhrenkatheter (6), der ein unmittelbares Segment (61), das in dem hindurchgehenden Loch (516) angeordnet ist und das sich der ersten Achse entlang erstreckt, um mit der Durchführung (513) zu kommunizieren, und ein distales Segment (62) umfasst, das sich von dem unmittelbaren Segment (61) der ersten Achse entlang erstreckt, um von der umgebenden Spitzenwand (512) nach außen herauszuragen;
eine Trommel (2, 9), die äußere und innere umgebende Trommelwandflächen (221, 222) aufweist, die einander gegenüberliegen und eine zweite Achse umgeben, wobei die innere umgebende Trommelwandfläche (222) einen Durchgang (21) umschließt, der offene nach vorne und nach hinten gerichtete Enden (223, 224, 931, 932) aufweist, die einander gegenüberliegend in Längsrichtung parallel zur zweiten Achse angeordnet sind, wobei die innere umgebende Trommelwandfläche (222) ein Segment von größerem Durchmesser (212, 92) und ein Segment von kleinerem Durchmesser (211, 91) umfasst, die hintere bzw. vordere Durchgangswege umschließen und die unmittelbar an den offenen nach hinten bzw. nach vorne gerichteten Enden (224, 223, 932, 931) angeordnet sind, um einen umgebenden Schulterabschnitt (213) zwischen dem Segment von größerem Durchmesser (212, 92) und dem Segment von kleinerem Durchmesser (211, 91) zu bilden, wobei die äußere umgebende Trommelwandfläche (221) eine vordere umgebende Region (2211) aufweist, die unmittelbar an dem offenen nach vorne gerichteten Ende (223, 931) gelegen ist und die mit Bezug auf die innere Buchsenwandfläche befestigt ist, was zu einem Zusammentreffen der ersten Achse mit der zweiten Achse führt;
eine Nadelkanüle (32), die eine Spitze und fixierte Enden (321, 322), die einander gegenüberliegen, und ein mittleres Segment (323) umfasst, das zwischen die Spitze und die fixierten Enden (321, 322) eingeschoben ist;
einen röhrenförmigen Nadelsitz (31), der in dem Durchgang (21) aufgenommen ist und sich der zweiten Achse entlang erstreckt, wobei der röhrenförmige Nadelsitz (31) Folgendes umfasst:
ein Nabenende (313), das zum Befestigen des fixierten Endes (322) angeordnet ist,
eine umgebende eingerastete Wand (311), die sich von dem Nabenende (313) der zweiten Achse entlang erstreckt und die einen Axialweg umschließt, um das fixierte Ende (322) unterzubringen oder damit zu kommunizieren, wobei die umgebende eingerastete Wand (311) eine äußere eingerastete Wandfläche aufweist, die die zweite Achse umgibt und die das Segment von kleinerem Durchmesser (211, 91) einrastet und mit Bezug auf dieses um die zweite Achse zwischen eingerasteten und freigegebenen Positionen rotierbar ist, wobei die umgebende eingerastete Wand (311) unbeweglich und mit Bezug auf das Segment von kleinerem Durchmesser (211, 91) der zweiten Achse entlang jeweils beweglich ist und
ein Verankerungssegment (312), das ein axiales hindurchgehendes Loch (3121) umschließt, um mit dem Durchgang (21) zu kommunizieren, und das sich außerhalb des vorderen Durchgangswegs von der umgebenden eingerasteten Wand (311) aus erstreckt, wobei das Verankerungssegment (312) am Verankerungsende (3122) endet, das mit der umgebenden eingerasteten Wand (311) rotiert wird, um sich von einer eingeklinkten Position, die dem umgebenden Schulterabschnitt (213) näher gelegen ist, zu einer eingedrückten Position zu bewegen, die von dem umgebenden Schulterabschnitt (213) entfernt ist, wenn die umgebende eingerastete Wand (311), im Uhrzeigersinn von der zwischeneingerasteten Position zur freigegebenen Position gedreht wird; und
einen Stößel (4), der in dem Durchgang (21) aufgenommen ist, derart, dass der Stößel (4) in einer Anwendungsposition dem Segment von größerem Durchmesser (212, 92) in Längsrichtung entlang bewegbar ist und derart, dass der Stößel (4) in einer Anordnungsposition dem Segment von größerem Durchmesser (212, 92) entlang unbeweglich, jedoch mit Bezug auf dieses rotierbar ist; wobei der Stößel (4) Folgendes umfasst:
einen Stößelkörper (42), der eine obere Endwand (421), die so angeordnet ist, dass sie den umgebenden Schulterabschnitt (213) konfrontiert, und eine untere Endwand (422) umfasst, die der oberen Endwand (421) in Längsrichtung gegenüberliegt, wobei die untere Endwand (422) sich von dem offenen nach hinten gerichteten Ende (224, 932) nach außen erstreckt, um die Bewegung und Rotation des Stößels (4) zu gestatten, wobei die obere Endwand (421) einen inneren peripheren Kantenabschnitt (424) aufweist, der die zweite Achse umgibt und der einen Hohlraum (41) darin definiert, wobei der Hohlraum (41) sich der zweiten Achse entlang und auf die untere Endwand (422) zu erstreckt und Fluid unter reduziertem Druck enthält und
ein Versiegelungsteil (43), das Folgendes umfasst
einen verankerten Abschnitt (432), der so angeordnet ist, dass er in das Verankerungsende (3122) derart einrastbar ist, dass, wenn der Stößel (4) sich in der Anordnungsposition befindet und wenn die umgebende eingerastete Wand (311) sich in der zwischengerasteten Position befindet, die Rotation des Stößelkörpers (42) im Uhrzeigersinn mit Bezug auf das Segment von größerem Durchmesser (212, 92) die umgebende eingerastete Wand (311) dazu bringt, sich von der zwischengerasteten Position zur freigegebenen Position zu drehen, wobei das Verankerungsende (3122) von der eingeklinkten Position zur eingedrückten Position bewegt wird, und
einen Versiegelungsabschnitt (434), der versiegelnd am inneren peripheren Kantenabschnitt (424) einer Versiegelungslinie entlang befestigt ist, um das Fluid in dem Hohlraum (41) aufzufangen, wobei die Versiegelungslinie derart konfiguriert ist, dass die Bewegung des Verankerungsendes (3122) von der eingeklinkten Position zur eingedrückten Position zum Eindrücken des verankerten Abschnitts (432) führt, um die Versiegelungslinie zu zerreißen, wodurch das Versiegelungsteil (43) vom Stößelkörper (42) freigegeben wird, so dass der röhrenförmige Nadelsitz (31) zusammen mit der Nadelkanüle (32) **durch** das Versiegelungsteil (43) gezogen wird, das in den Hohlraum (41) aufgrund eines Druckunterschieds zwischen dem reduzierten Druck und der Umgebungsluft gezogen wird, die **durch** die Kommunikationsöffnung (514) eingeführt wird, wodurch das Zurückziehen der Nadelkanüle (32) aus dem Röhrenkatheter (6) in den Hohlraum (41) gestattet wird.

2. Gerät zum Insertieren eines intravenösen Katheters nach Anspruch 1, **dadurch gekennzeichnet, dass** die vordere umgebende Region (2211) der äußeren umgebenden Trommelwandfläche (221) der Trommel (2) in das Insertionsloch (517) derart eingeführt wird, dass sie an der inneren Buchsenwandfläche der Buchsenwand (511) der Katheternabe (5) anstößt.

3. Gerät zum Insertieren eines intravenösen Katheters nach Anspruch 2, **dadurch gekennzeichnet, dass** der Stößelkörper (42) eine äußere Stößelwandfläche (423) aufweist, die die zweite Achse umgibt, wobei das Gerät zum Insertieren eines intravenösen Katheters des Weiteren erste Außen- und Innenschraubengewindesegmente (425, 225) umfasst, die jeweils an der äußeren Stößelwandfläche (423) und dem Segment von größerem Durchmesser (212, 92) angeordnet sind, um den Stößelkörper (42) davon abzuhalten, sich dem Segment von größerem Durchmesser (212, 92) entlang zu verschieben, jedoch die Rotation des Stößelkörpers (42) im Uhrzeigersinn mit Bezug auf das Segment von größerem Durchmesser (212, 92) in der Anordnungsposition zu gestatten.

4. Gerät zum Insertieren eines intravenösen Katheters nach Anspruch 3, des Weiteren **gekennzeichnet durch** zweite Außen-und Innenschraubengewindesegmente (314, 226), die jeweils an der äußeren eingerasteten Wandfläche und dem Segment von kleinerem Durchmesser (211, 91) angeordnet sind, um die Rotation der äußeren eingerasteten Wandfläche mit Bezug auf das Segment von kleinerem Durchmesser (211, 91) zu gestatten.

5. Gerät zum Insertieren eines intravenösen Katheters nach Anspruch 4, **dadurch gekennzeichnet, dass** das Verankerungssegment (312) aus einem Material besteht, das lichtdurchlässig ist, um das Betrachten von Flüssigkeit, die durch das axiale hindurchgehende Loch (3121) hindurchfließt, zu gestatten.

6. Gerät zum Insertieren eines intravenösen Katheters nach Anspruch 5, **dadurch gekennzeichnet, dass** das Verankerungssegment (312) so konfiguriert ist, dass es einen vergrößerten Blick von Blut in dem axialen hindurchgehenden Loch (3121) bereitstellt.

7. Gerät zum Insertieren eines intravenösen Katheters nach Anspruch 6, **dadurch gekennzeichnet, dass** das Versiegelungsteil (43) aus einem Elastomermaterial gebildet ist.

8. Gerät zum Insertieren eines intravenösen Katheters nach Anspruch 7, **dadurch gekennzeichnet, dass** der verankerte Abschnitt (432) eine innere umgebende Wand aufweist, die sich der zweiten Achse entlang erstreckt, um eine Aussparung (431) zu umschließen, die so konfiguriert ist, dass sie das Verankerungsende (3122) unterbringt, wobei das Gerät zum Insertieren eines intravenösen Katheters des Weiteren dritte Außen- und Innenschraubengewindesegmente (315, 433) umfasst, die jeweils an dem Verankerungsende (3122) und der inneren umgebenden Wand angeordnet sind, um zu gestatten, dass der verankerte Abschnitt (432) dazu gebracht wird, sich in das Verankerungsende (3122) durch Rotation der dritten Außen- und Innenschraubengewindesegmente (315, 433) mit Bezug aufeinander einzurasten.

9. Gerät zum Insertieren eines intravenösen Katheters nach Anspruch 8, des Weiteren **gekennzeichnet durch** einen umgebenden Vorsprung (4241), der so angeordnet ist, dass er sich von dem inneren peripheren Kantenabschnitt (424) in Radialrichtung und auf die zweite Achse zu so erstreckt, dass er sich in Reibungskontakt mit dem Versiegelungsabschnitt (434) befindet, wodurch die Versiegelungslinie festgesetzt wird.

10. Gerät zum Insertieren eines intravenösen Katheters nach Anspruch 9, des Weiteren **gekennzeichnet durch** einen Fängerabschnitt (426), der auf dem inneren peripheren Kantenabschnitt (424) unmittelbar an der unteren Endwand (422) angeordnet und konfiguriert ist, um den röhrenförmigen Nadelsitz (31) und die Nadelkanüle (32) daran zu hindern, auf die untere Endwand (422) einzuwirken.

11. Gerät zum Insertieren eines intravenösen Katheters nach Anspruch 1, **dadurch gekennzeichnet, dass** die innere Muffenwandfläche der Muffenwand (511) einen Grenzbereich (519) umschließt, der die erste Achse umgibt, und eine Wiedereintrittsöffnung (515) aufweist, die sich radial durch die Buchsenwand (511) erstreckt, um mit dem Insertionsloch (517) zu kommunizieren, und die sich der Kommunikationsöffnung (514) mit Bezug auf den Grenzbereich (519) gegenüberliegend befindet, wobei das Gerät zum Insertieren eines intravenösen Katheters des Weiteren Folgendes umfasst:
ein Barriereteil (54), das die innere Buchsenwandfläche am Grenzbereich (519) einrastet; und
einen röhrenförmigen Einsatz (53), der ein vorderes Halsende (534), das mit dem Barriereteil (54) verbunden ist und das einen hindurchgehenden Weg (531) der ersten Achse entlang umschließt, und ein hinteres aufgeweitetes Ende (532) aufweist, das sich von dem vorderen Halsende (534) der ersten Achse entlang erstreckt und das eine innere angestoßene Fläche (536) aufweist, wobei die innere angestoßene Fläche (536) eine erste Achse umgibt und einen Fluidweg (535) umschließt, der sich zum Kommunizieren mit der Wiedereintrittsöffnung (515) erstreckt, wobei die innere angestoßene Fläche (536) die vordere umgebende Region (2211) der äußeren umgebenden Trommelwandfläche (221) der Trommel (2) einrastet, wodurch der hindurchgehende Weg (531) auf den Durchgang (21) der Trommel (2) so ausgerichtet wird, dass es der Nadelkanüle (32) gestattet wird, sich durch den hindurchgehenden Weg (531) und außerhalb des Röhrenkatheters (6) zu erstrecken.

12. Intravenöses Kathetergerät zur Verwendung mit einer wegwerfbaren Spritze, die Folgendes umfasst:
eine Trommel (2, 9), die eine innere umgebende Trommelwandfläche (222) aufweist, die einen Durchgang (21) umschließt, der sich in einer Längsrichtung erstreckt und der offene nach vorne und nach hinten gerichtete Enden (223, 224, 931, 932), die in Längsrichtung einander gegenüberliegen, und eine äußere umgebende Trommelwandfläche (221) aufweist, die eine vordere umgebende Region (2211) aufweist, die unmittelbar am offenen nach vorne gerichteten Ende (223, 931) gelegen ist,
eine Nadelkanüle (32), die zurückziehbar in dem offenen nach vorne gerichteten Ende (223, 931) aufgenommen ist, und
einen Stößel (4), der in dem Durchgang (21) so aufgenommen ist, dass er in Längsrichtung beweglich ist, der sich von dem offenen nach hinten gerichteten Ende (224, 932) nach außen so erstreckt, dass er manuell bedienbar ist, der einen Hohlraum (41) aufweist, der sich in Längsrichtung erstreckt und ein Fluid unter reduziertem Druck enthält, und der mit der Nadelkanüle (32) innerhalb des Durchgangs (21) so gekoppelt ist, dass die Nadelkanüle (32) in den Hohlraum (41) zurückgezogen wird, sobald die Nadelkanüle (32) einer Saugkraft unterworfen wird, die als Resultat eines Druckunterschieds zwischen der Umgebungsluft und dem reduzierten Druck auftritt, wobei das intravenöse Kathetergerät ***gekennzeichnet ist durch**:*
eine Katheternabe (5), die Folgendes umfasst:
eine umgebende Spitzenwand (512), die eine erste Achse umgibt und ein hindurchgehendes Loch (516) umschließt, das sich der ersten Achse entlang erstreckt.
eine Buchsenwand (511), die der umgebenden Spitzenwand (512) der ersten Achse entlang gegenüberliegt und die eine innere Buchsenwandfläche aufweist, die die erste Achse umgibt und die ein Insertionsloch (517) umschließt, das größer als das hindurchgehende Loch (516) ist , wobei die innere Buchsenwandfläche geeignet ist, mit Bezug auf die vordere umgebende Region (2211) der äußeren umgebenden Trommelwandfläche (221) der Trommel (2) befestigt zu werden, um den Durchgang (21) in Ausrichtung mit dem hindurchgehenden Loch (516) zu bringen und
eine Zwischenröhrenwand (51), die zwischen die umgebende Spitzenwand (512) und die Buchsenwand (511) eingeschoben ist, die eine Durchführung (513) umschließt, die das Insertionsloch (517) mit dem hindurchgehenden Loch (516) in Kommunikation bringt und die geeignet ist, dass die Nadelkanüle (32) hindurchgeht, wenn die innere Buchsenwandfläche mit Bezug auf die vordere umgebende Region (2211) der äußeren umgebenden Trommelwandfläche (221) der Trommelwand (2) befestigt ist und die eine Kommunikationsöffnung (514) aufweist, die sich hindurch erstreckt, um die Durchführung (513) mit der Umgebungsluft in Kommunikation zu bringen; und
einen Röhrenkatheter (6), der ein unmittelbares Segment (61), das in dem hindurchgehenden Loch (516) angeordnet ist und das sich der ersten Achse entlang erstreckt, um mit der Durchführung (513) zu kommunizieren, und ein distales Segment (62) umfasst, das sich von dem unmittelbaren Segment (61) der ersten Achse entlang erstreckt, um von der umgebenden Spitzenwand (512) nach außen vorzuspringen, und das geeignet ist, um auf der Nadelkanüle (32) aufgebuchst zu werden und um zu gestatten, dass die Nadelkanüle (32) sich nach außen von derselben erstreckt, wenn die Nadelkanüle (32) dazu gebracht wird, **durch** die Durchführung (513) hindurchzugehen;
wobei die Zwischenröhrenwand (51) eine innere Röhrenwandfläche, um die Durchführung (513) zu umschließen, und eine äußere Röhrenwandfläche der inneren Röhrenwandfläche in Radialrichtungen gegenüberliegend aufweist, wobei die innere Röhrenwandfläche eine Rückhalteschulter (518) mit der umgebenden Spitzenwand (512) bildet, wobei die Rückhalteschulter (518) die Durchführung (513) der Achse entlang konfrontiert, wobei das unmittelbare Segment (61) sich der Achse entlang erstreckt, um nach außen aus dem hindurchgehenden Loch (516) herauszuragen und am Flanschabschnitt (611) zu enden, der an der Rückhalteschulter (518) anstößt und **durch** diese gehalten wird;
wobei die Kommunikationsöffnung (514) sich radial **durch** die inneren und äußeren Röhrenwandflächen hindurch erstreckt;
wobei die innere Buchsenwandfläche einen Grenzbereich (519) umschließt, der die Achse umgibt, und eine Wiedereintrittsöffnung (515) aufweist, die sich radial durch die Buchsenwand (511) erstreckt, um mit dem Insertionsloch (517) zu kommunizieren und die der Kommunikationsöffnung (514) mit Bezug auf den Grenzbereich (519) gegenüberliegt;
wobei das intravenöse Kathetergerät des Weiteren Folgendes umfasst:
ein Barriereteil (54), das die innere Buchsenwandfläche am Grenzbereich (519) einrastet; und
einen röhrenförmigen Einsatz (53), der ein vorderes Halsende (534), das mit dem Barriereteil (54) verbunden ist und das einen hindurchgehenden Weg (531) der ersten Achse entlang umschließt, und ein hinteres aufgeweitetes Ende (532) aufweist, das sich von dem vorderen Halsende (534) der ersten Achse entlang erstreckt und das eine innere angestoßene Fläche (536) aufweist, wobei die innere angestoßene Fläche (536) die Achse umgibt und einen Fluidweg (535) umschließt, der sich zum Kommunizieren mit der Wiedereintrittsöffnung (515) erstreckt, wobei die innere angestoßene Fläche (536) geeignet ist, die vordere umgebende Region (2211) der äußeren umgebenden Trommelwandfläche (221) der Trommel (2) einzurasten, wodurch der hindurchgehende Weg (531) auf den Durchgang (21) der Trommel (2) so ausgerichtet wird, dass es der Nadelkanüle (32) gestattet wird, sich **durch** den hindurchgehenden Weg (531) und außerhalb des Röhrenkatheters (6) zu erstrecken.

13. Intravenöses Kathetergerät nach Anspruch 12, **dadurch gekennzeichnet, dass** das Barriereteil (54) aus einem Elastomermaterial gebildet ist.

14. Intravenöses Kathetergerät zur Verwendung mit einer wegwerfbaren Spritze, die Folgendes umfasst:
eine Trommel (2, 9), die eine innere umgebende Trommelwandfläche (222) aufweist, die einen Durchgang (21) umschließt, der sich in einer Längsrichtung erstreckt und der offene nach vorne und nach hinten gerichtete Enden (223, 224, 931, 932), die in Längsrichtung einander gegenüberliegen, und eine äußere umgebende Trommelwandfläche (221) aufweist, die eine vordere umgebende Region (2211) aufweist, die unmittelbar am offenen nach vorne gerichteten Ende (223, 931) gelegen ist,
eine Nadelkanüle (32), die zurückziehbar in dem offenen nach vorne gerichteten Ende (223, 931) aufgenommen ist, und
einen Stößel (4), der in dem Durchgang (21) so aufgenommen ist, dass er in Längsrichtung beweglich ist, der sich von dem offenen nach hinten gerichteten Ende (224, 932) nach außen so erstreckt, dass er manuell bedienbar ist, der einen Hohlraum (41) aufweist, der sich in Längsrichtung erstreckt und ein Fluid unter reduziertem Druck enthält, und der mit der Nadelkanüle (32) innerhalb des Durchgangs (21) so gekoppelt ist, dass die Nadelkanüle (32) in den Hohlraum (41) zurückgezogen wird, sobald die Nadelkanüle (32) einer Saugkraft unterworfen wird, die als Resultat eines Druckunterschieds zwischen der Umgebungsluft und dem reduzierten Druck auftritt, wobei das intravenöse Kathetergerät ***gekennzeichnet ist durch**:*
eine Katheternabe (5), die Folgendes umfasst
eine umgebende Spitzenwand (512), die eine erste Achse umgibt und ein hindurchgehendes Loch (516) umschließt, das sich der ersten Achse entlang erstreckt,
eine Buchsenwand (511), die der umgebenden Spitzenwand (512) der ersten Achse entlang gegenüberliegt und die eine innere Buchsenwandfläche aufweist, die die erste Achse umgibt und die ein Insertionsloch (517) umschließt, das größer als das hindurchgehende Loch (516) ist, wobei die innere Buchsenwandfläche geeignet ist, mit Bezug auf die vordere umgebende Region (2211) der äußeren umgebenden Trommelwandfläche (221) der Trommel (2) befestigt zu werden, um den Durchgang (21) in Ausrichtung mit dem hindurchgehenden Loch (516) zu bringen und
eine Zwischenröhrenwand (51), die zwischen die umgebende Spitzenwand (512) und die Buchsenwand (511) eingeschoben ist, die eine Durchführung (513) umschließt, die das Insertionsloch (517) mit dem hindurchgehenden Loch (516) in Kommunikation bringt und die geeignet ist, dass die Nadelkanüle (32) hindurchgeht, wenn die innere Buchsenwandfläche mit Bezug auf die vordere umgebende Region (2211) der äußeren umgebenden Trommelwandfläche (221) der Trommelwand (2) befestigt ist und die eine Kommunikationsöffnung (514) aufweist, die sich hindurch erstreckt, um die Durchführung (513) mit der Umgebungsluft in Kommunikation zu bringen; und
einen Röhrenkatheter (6), der ein unmittelbares Segment (61), das in dem hindurchgehenden Loch (516) angeordnet ist und das sich der Achse entlang erstreckt, um mit der Durchführung (513) zu kommunizieren, und ein distales Segment (62) umfasst, das sich von dem unmittelbaren Segment (61) der Achse entlang erstreckt, um von der umgebenden Spitzenwand (512) nach außen vorzuspringen, und das geeignet ist, um auf der Nadelkanüle (32) aufgebuchst zu werden und um zu gestatten, dass die Nadelkanüle (32) sich nach außen von derselben erstreckt, wenn die Nadelkanüle (32) dazu gebracht wird, **durch** die Durchführung (513) hindurchzugehen;
wobei die Zwischenröhrenwand (51) eine innere Röhrenwandfläche, um die Durchführung (513) zu umschließen, und eine äußere Röhrenwandfläche der inneren Röhrenwandfläche in Radialrichtungen gegenüberliegend aufweist, wobei die innere Röhrenwandfläche eine Rückhalteschulter (518) mit der umgebenden Spitzenwand (512) bildet, wobei die Rückhalteschulter (518) die Durchführung (513) der Achse entlang konfrontiert, wobei das unmittelbare Segment (61) sich der Achse entlang erstreckt, um nach außen aus dem hindurchgehenden Loch (516) herauszuragen und am Flanschabschnitt (611) zu enden, der an der Rückhalteschulter (518) anstößt und **durch** diese gehalten wird;
wobei die Kommunikationsöffnung (514) sich radial **durch** die inneren und äußeren Röhrenwandflächen hindurch erstreckt;
wobei die innere Buchsenwandfläche einen Grenzbereich umschließt, der die Achse umgibt;
wobei das intravenöse Kathetergerät des Weiteren Folgendes umfasst:
ein Barriereteil (54), das die innere Buchsenwandfläche am Grenzbereich einrastet; und
einen röhrenförmigen Einsatz (55), der ein vorderes Halsende (554), das mit dem Barriereteil (54) verbunden ist und das einen hindurchgehenden Weg der Achse entlang umschließt, und ein hinteres aufgeweitetes Ende (552) aufweist, das sich von dem vorderen Halsende (554) der Achse entlang erstreckt, wobei das hintere aufgeweitete Ende (552) eine innere angestoßene Fläche, die die Achse umgibt und einen Fluidweg (551) umschließt, und eine Wiedereintrittsöffnung (555) aufweist, die sich radial erstreckt, um mit dem Fluidweg (551) zu kommunizieren und die der Kommunikationsöffnung (514) mit Bezug auf den Grenzbereich gegenüberliegt, wobei die innere angestoßene Fläche des hinteren aufgeweiteten Endes (552) geeignet ist, die vordere umgebende Region (2211) der äußeren umgebenden Trommelwandfläche (221) der Trommel (2) einzurasten, wodurch der hindurchgehende Weg auf den Durchgang (21) der Trommel (2) so ausgerichtet wird, dass es der Nadelkanüle (32) gestattet wird, sich **durch** den hindurchgehenden Weg (531) und außerhalb des Röhrenkatheters (6) zu erstrecken.

## Revendications

1. Dispositif d'insertion d'un cathéter intraveineux, **caractérisé par** :
un pavillon de cathéter (5) comprenant
une paroi enveloppant l'extrémité (512) qui entoure un premier axe et délimite un orifice de passage (516) qui se prolonge le long du premier axe,
une paroi de la gaine (511) qui est à l'opposé de ladite paroi enveloppant l'extrémité (512) le long du premier axe et dont une surface de la paroi interne de la gaine entoure le premier axe et délimite un orifice d'insertion (517) plus gros que ledit orifice de passage (516), et
une paroi tubulaire intermédiaire (51) interposée entre ladite paroi entourant l'extrémité (512) et ladite paroi de la gaine (511), qui délimite un conduit (513) mettant en communication ledit orifice d'insertion (517) avec ledit orifice de passage (516) et présente une embouchure de communication (514) qui se prolonge au travers pour mettre en communication ledit conduit (513) avec l'air ambiant ;
un cathéter tubulaire (6) comportant un segment proximal (61) qui est disposé dans ledit orifice de passage (516) et se prolonge le long du premier axe et communique avec ledit conduit (513), et un segment distal (62) qui se prolonge à partir dudit segment proximal (61) le long du premier axe et se projette en direction de l'extérieur de ladite paroi entourant l'extrémité (512) ;
un corps (2, 9) présentant des surfaces de la paroi externe et interne enveloppant le corps (221, 222) qui sont à l'opposé l'une de l'autre et entourent un second axe, ladite surface de la paroi interne enveloppant le corps (222) délimitant un passage (21) dont les extrémités antérieure et postérieure sont ouvertes (223, 224, 931, 932) et disposées à l'opposé l'une de l'autre dans une direction longitudinale parallèle au second axe, ladite surface de la paroi interne enveloppant le corps (222) comportant un segment de diamètre plus large (212, 92) et un segment de diamètre plus faible (211, 91) qui délimitent des passages postérieur et antérieur, respectivement, et qui sont disposés en amont desdites extrémités postérieure et antérieure ouvertes (224, 223, 932, 931), respectivement, pour former une portion d'épaulement enveloppante (213) entre ledit segment de diamètre plus large (212, 92) et ledit segment de diamètre plus faible (211, 91), ladite surface de la paroi externe enveloppant le corps (221) présentant une région antérieure enveloppante (2211) en amont de ladite extrémité antérieure ouverte (223, 931) et étant fixée par rapport à surface de la paroi interne de la gaine enveloppante, produisant ainsi une coïncidence entre le premier et le second axes ;
une canule à aiguille (32) comportant des extrémités antérieure et postérieure fixes (321, 322) à l'opposé l'une de l'autre et un segment intermédiaire (323) interposé entre lesdites extrémités antérieure et postérieure fixes (321, 322) ;
une embase d'aiguille tubulaire (31) abritée dans ledit passage (21) et se prolongeant le long du second axe, ladite embase d'aiguille tubulaire (31) comportant
une extrémité femelle (313) disposée de façon à arrimer ladite extrémité fixe (322),
une paroi accouplée enveloppante (311) qui se prolonge à partir de ladite extrémité femelle (313) le long du second axe et délimite une voie axiale permettant l'adaptation ou la communication avec ladite extrémité fixe (322), ladite paroi accouplée enveloppante (311) présentant une surface accouplée de la paroi externe qui entoure le second axe et qui est accouplée au segment de plus faible diamètre (211, 91) et rotative par rapport à celui-ci autour du second axe entre des positions d'accouplement réciproque et de relâchement, où ladite paroi accouplée enveloppante (311) est inamovible et amovible par rapport au dit segment de plus faible diamètre (211, 91) le long du second axe, respectivement, et
un segment d'ancrage (312) qui délimite un orifice de passage axial (3121) de communiquer avec ledit passage (21) et qui se prolonge en direction de l'extérieur dudit passage antérieur à partir de ladite paroi accouplée enveloppante (311), ledit segment d'ancrage (312) se terminant à une extrémité d'ancrage (3122) qui tourne avec ladite paroi accouplée enveloppante (311) pour passer d'une position connectée qui est plus proche de ladite portion d'épaulement enveloppante (213) à une position abaissée qui est éloignée de ladite portion d'épaulement enveloppante (213) quand ladite paroi accouplée enveloppante (311) est tournée dans le sens des aiguilles d'une montre de la position d'accouplement réciproque à la position de relâchement ; et
un piston (4) abrité dans ledit passage (21) de sorte que dans une position d'utilisation, ledit piston (4) est déplaçable le long dudit segment de diamètre plus large (212, 92) en direction longitudinale et de sorte que dans une position d'évacuation, ledit piston (4) est inamovible le long du segment de diamètre plus large mais rotatif par rapport à celui-ci (212, 92), ledit piston (4) comportant
un corps de piston (42) qui comporte une paroi à l'extrémité supérieure (421) disposée de manière à être en confrontation avec la portion d'épaulement enveloppante (213) et une paroi à l'extrémité inférieure (422) à l'opposé de ladite paroi à l'extrémité supérieure (421) en direction longitudinale, ladite paroi à l'extrémité inférieure (422) se prolongeant en direction de l'extérieur de ladite extrémité postérieure ouverte (224, 932) pour permettre le déplacement et la rotation dudit piston (4), ladite paroi à l'extrémité supérieure (421) présentant une portion à bordure périphérique interne (424) qui entoure le second axe et qui définit une cavité (41) à l'intérieur, ladite cavité (41) se prolongeant le long du second axe en direction de ladite paroi à l'extrémité inférieure (422) et contenant un liquide à une pression réduite, et
un élément d'étanchéité (43) comportant
une portion ancrée (432) disposée de façon à pouvoir être accouplée à ladite extrémité d'ancrage (3122) de sorte que quand ledit piston (4) est dans la position d'évacuation et quand ladite paroi accouplée enveloppante (311) est dans la position d'accouplement réciproque, la rotation du corps dudit piston (42) dans le sens des aiguilles d'une montre par rapport au dit segment de diamètre plus large (212, 92) conduit ladite paroi accouplée enveloppante (311) à tourner de la position d'accouplement réciproque à la position de relâchement, déplaçant ainsi ladite extrémité d'ancrage (3122) de la position connectée à la position abaissée, et
une portion d'étanchéité (434) qui est raccordée de manière étanche à ladite à bordure périphérique interne (424) le long d'une ligne de scellement de façon à piéger ledit liquide dans ladite cavité (41), la ligne de scellement étant configurée de façon à ce que le déplacement de ladite extrémité d'ancrage (3122) de la position connectée à la position abaissée résulte en un abaissement de ladite portion d'ancrage (432) conduisant à une déchirure de ladite ligne de scellement, libérant ainsi ledit élément d'étanchéité (43) du corps dudit piston (42) de sorte que ladite embase d'aiguille tubulaire (31) ainsi que ladite canule à aiguille (32) seront tirées par ledit élément d'étanchéité (43) qui est aspiré dans ladite cavité (41) sous l'effet d'une différence de pression entre la pression réduite et l'air ambiante introduit par l'intermédiaire de ladite embouchure de communication (514), ce qui permet la rétraction de ladite canule à aiguille (32) à partir dudit cathéter tubulaire (6) dans ladite cavité (41).

2. Dispositif d'insertion d'un cathéter intraveineux selon la revendication 1, **caractérisé en ce que** la région antérieure enveloppante (2211) de ladite surface de la paroi externe (221) enveloppant ledit corps (2) est insérée dans ledit orifice d'insertion (517) de façon à venir en aboutement contre ladite surface de la paroi interne de ladite paroi de la gaine (511) du pavillon dudit cathéter (5).

3. Dispositif d'insertion d'un cathéter intraveineux selon la revendication 2, **caractérisé en ce que** le corps dudit piston (42) présente une surface de la paroi externe du piston (423) qui entoure le second axe, ledit dispositif d'insertion d'un cathéter intraveineux comprenant également des premiers segments à filetage vissé mâle et femelle (425, 225) disposés sur ladite surface de la paroi externe du piston (423) et sur ledit segment à diamètre plus large (212, 92), respectivement, de façon à empêcher le déplacement du corps dudit piston (42) le long dudit segment de diamètre plus large (212, 92) tout en permettant une rotation du corps dudit piston (42) dans le sens des aiguilles d'une montre par rapport au dit segment de diamètre plus large (212, 92) dans la position d'évacuation.

4. Dispositif d'insertion d'un cathéter intraveineux selon la revendication 3, également **caractérisé en ce que** les seconds segments à filetage vissé mâle et femelle (314, 226) sont disposés sur ladite surface de la paroi externe accouplée et sur ledit segment à diamètre plus faible (211, 91), respectivement, de façon à permettre une rotation de ladite surface de la paroi externe accouplée par rapport au dit segment de diamètre plus faible (211, 91).

5. Dispositif d'insertion d'un cathéter intraveineux selon la revendication 4, **caractérisé en ce que** le segment d'ancrage (312) est composé d'un matériau qui est translucide de façon à permettre une visualisation de l'écoulement du liquide au travers dudit orifice de passage axial (3121).

6. Dispositif d'insertion d'un cathéter intraveineux selon la revendication 5, **caractérisé en ce que** ledit segment d'ancrage (312) est configuré de manière à produire un grossissement optique du sang dans ledit orifice de passage axial (3121).

7. Dispositif d'insertion d'un cathéter intraveineux selon la revendication 6, **caractérisé en ce que** ledit élément d'étanchéité (43) est composé d'un matériau élastomère.

8. Dispositif d'insertion d'un cathéter intraveineux selon la revendication 7, **caractérisé en ce que** ladite portion ancrée (432) présente une paroi interne enveloppante qui se prolonge le long du second axe et délimite un évidement (431) configuré pour s'adapter à ladite extrémité d'ancrage (3122), ledit dispositif d'insertion d'un cathéter intraveineux comprenant également des troisièmes segments à filetage vissé mâle et femelle (315, 433) disposés sur ladite extrémité d'ancrage (3122) et sur ladite paroi interne enveloppante, respectivement, de façon à permettre à ladite portion ancrée (432) d'être amenée à ladite extrémité d'ancrage (3122) et de s'accoupler à celle-ci sous l'effet d'une rotation des troisièmes segments à filetage vissé mâle et femelle (315, 433) l'un par rapport à l'autre.

9. Dispositif d'insertion d'un cathéter intraveineux selon la revendication 8, également **caractérisé par** une saillie enveloppante (4241) disposée de façon à se prolonger à partir de ladite portion à bordure périphérique interne (424) dans des directions radiales et vers le second axe de sorte à se trouver en contact frictionnel avec ladite portion d'étanchéité (434) et à établir ainsi la ligne de scellement.

10. Dispositif d'insertion d'un cathéter intraveineux selon la revendication 9, également **caractérisé par** une portion de retenue (426) disposée sur ladite portion à bordure périphérique interne (424) en amont de ladite paroi à l'extrémité inférieure (422) et configurée de façon à empêcher ladite embase d'aiguille tubulaire (31) et ladite canule à aiguille (32) de heurter ladite paroi à l'extrémité inférieure (422).

11. Dispositif d'insertion d'un cathéter intraveineux selon la revendication 1, **caractérisé par** en ce que la surface de la paroi interne de la gaine (511) délimite une zone de séparation (519) qui entoure le premier axe et présente un embouchure de réentrée (515) qui se prolonge radialement au travers de ladite paroi de la gaine (511) et communique avec ledit orifice d'insertion (517) et qui est à l'opposé dudit embouchure de communication (514) par rapport à ladite zone de séparation (519), ledit dispositif d'insertion d'un cathéter intraveineux comprenant également :
un élément barrière (54) qui s'accouple à ladite surface de la paroi interne de la gaine au niveau de ladite zone de séparation (519) ; et
un élément rapporté tubulaire (53) qui présente une extrémité antérieure rétrécie (534) raccordée au dit élément barrière (54) et délimite un passage (531) le long du premier axe, et une extrémité postérieure évasée (532) qui se prolonge à partir de ladite extrémité antérieure rétrécie (534) le long du premier axe et présente une surface interne aboutée (536), ladite surface interne aboutée (536) entourant le premier axe et délimitant un passage liquidien (535) qui se prolonge et communique avec ledit embouchure de réentrée (515), et ladite surface interne aboutée (536) s'accouplant à ladite région antérieure enveloppante (2211) de ladite surface de la paroi externe (221) enveloppant ledit corps (2) et produisant ainsi un alignement entre ladite voie de passage direct (531) et ledit passage (21) dudit corps (2) afin de permettre l'extension de ladite canule à aiguille (32) au travers de ladite voie de passage direct (531) et en direction de l'extérieur dudit cathéter tubulaire (6).

12. Cathéter intraveineux conçu pour être utilisé avec une seringue jetable, qui comporte
un corps (2, 9) présentant une surface de paroi interne du corps (222) qui délimite un passage (21) qui se prolonge en une direction longitudinale et possède des extrémités antérieure et postérieure ouvertes (223, 224, 931, 932) à l'opposé l'une de l'autre dans la direction longitudinale et une surface de paroi externe du corps enveloppante (221) présentant une région antérieure enveloppante (2211) en amont de l'extrémité antérieure ouverte (223, 931),
une canule à aiguille (32) abritée de manière rétractable dans l'extrémité antérieure ouverte (223, 931), et
un piston (4) qui est abrité dans le passage (4) de façon à pouvoir être déplacé dans la direction longitudinale, se prolonge en direction de l'extérieur de l'extrémité postérieure ouverte (224, 932) de sorte qu'il est possible de le faire fonctionner manuellement, présente une cavité (41) qui se prolonge dans la direction longitudinale et contient un liquide à une pression réduite et est couplé à la canule à aiguille (32) à l'intérieur du passage (21) de sorte que la canule à aiguille (32) peut être rétractée dans la cavité (41) quand la canule à aiguille (32) est soumise à une force de succion produite en résultat d'une différence de pression entre l'air ambiant et la pression réduite, ledit cathéter intraveineux étant **caractérisé par** :
un pavillon de cathéter (5) comprenant
une paroi enveloppant l'extrémité (512) qui entoure un axe et délimite un orifice de passage (516) qui se prolonge le long de l'axe,
une paroi de la gaine (511) qui est à l'opposé de ladite paroi enveloppant l'extrémité (512) le long de l'axe et dont une surface de la paroi interne de la gaine entoure l'axe et délimite un orifice d'insertion (517) plus gros que ledit orifice de passage (516), ladite surface de la paroi interne de la gaine étant adaptée de façon à être arrimée par rapport à la région antérieure enveloppante (2211) de la surface de la paroi externe enveloppante (221) du corps (2) pour produire un alignement entre le passage (21) et ledit orifice de passage (516), et
une paroi tubulaire intermédiaire (51) interposée entre ladite paroi enveloppant l'extrémité (512) et ladite paroi de la gaine (511), qui délimite un conduit (513) mettant en communication ledit orifice d'insertion (517) avec ledit orifice de passage (516) et qui est adaptée de façon à ce que la canule à aiguille (32) passe au travers quand ladite surface de la paroi interne de la gaine est arrimée par rapport à la région antérieure enveloppante (2211) de la surface de la paroi externe enveloppante (221) du corps (2), et qui présente une embouchure de communication (514) qui se prolonge au travers pour mettre en communication ledit conduit (513) avec l'air ambiant ; et
un cathéter tubulaire (6) comportant un segment proximal (61) qui est disposé dans ledit orifice de passage (516) et se prolonge le long de l'axe et communique avec ledit conduit (513), et un segment distal (62) qui se prolonge à partir dudit segment proximal (61) le long de l'axe et se projette en direction de l'extérieur de ladite paroi enveloppant l'extrémité (512), et qui est adapté pour être glissé sur la canule à aiguille (32) et permettre une extension de la canule à aiguille (32) en direction de l'extérieur de celle-ci quand la canule à aiguille (32) est amenée à passer au travers dudit conduit (513) ;
ladite paroi tubulaire intermédiaire (51) présentant une surface de paroi tubulaire interne qui délimite ledit conduit (513) et une surface de paroi tubulaire externe à l'opposé de ladite surface de paroi tubulaire interne en directions radiales, ladite surface de paroi tubulaire interne formant un épaulement de retenue (518) avec ladite paroi enveloppant l'extrémité (512) et ledit épaulement de retenue (518) étant en confrontation avec ledit conduit (513) le long de l'axe, ledit segment proximal (61) se prolongeant le long de l'axe et se projetant en direction de l'extérieur dudit orifice de passage (516) et se terminant par une portion à brides (611) en aboutement contre et retenue par ledit épaulement de retenue (518) ;
ladite embouchure de communication (514) se prolongeant radialement au travers desdites surfaces de la paroi tubulaire interne et externe ;
ladite surface de la paroi interne de la gaine délimitant une zone de séparation (519) qui entoure l'axe et présente un orifice de réentrée (515) qui se prolonge radialement au travers de ladite paroi de la gaine (511) et communique avec ledit orifice d'insertion (517) et qui est à l'opposé dudit embouchure de communication (514) par rapport à ladite zone de séparation (519) ;
ledit cathéter intraveineux comprenant également :
un élément barrière (54) qui s'accouple à ladite surface de la paroi interne de la gaine au niveau de ladite zone de séparation (519) ; et
un élément rapporté tubulaire (53) qui présente une extrémité antérieure rétrécie (534) qui est raccordée au dit élément barrière (54) et délimite un passage (531) le long du premier axe, et une extrémité postérieure évasée (532) qui se prolonge à partir de ladite extrémité antérieure rétrécie (534) le long de l'axe et présente une surface interne aboutée (536), ladite surface interne aboutée (536) entourant l'axe et délimitant un passage liquidien (535) qui se prolonge et communique avec ledit orifice de réentrée (515), et ladite surface interne aboutée (536) étant adaptée pour s'accoupler à ladite région antérieure enveloppante (2211) de ladite surface de la paroi externe (221) enveloppant ledit corps (2) et produisant ainsi un alignement entre ladite voie de passage direct (531) et ledit passage (21) dudit corps (2) afin de permettre l'extension de ladite canule à aiguille (32) au travers de ladite voie de passage direct (531) et en direction de l'extérieur dudit cathéter tubulaire (6).

13. Dispositif d'insertion d'un cathéter intraveineux selon la revendication 12, **caractérisé en ce que** ledit élément barrière (54) est composé d'un matériau élastomère.

14. Cathéter intraveineux conçu pour être utilisé avec une seringue jetable, qui comporte
un corps (2, 9) présentant une surface de paroi interne du corps (222) qui délimite un passage (21) qui se prolonge en une direction longitudinale et possède des extrémités antérieure et postérieure ouvertes (223, 224, 931, 932) à l'opposé l'une de l'autre dans la direction longitudinale et une surface de paroi externe du corps enveloppante (221) présentant une région antérieure enveloppante (2211) en amont de l'extrémité antérieure ouverte (223, 931),
une canule à aiguille (32) abritée de manière rétractable dans l'extrémité antérieure ouverte (223, 931), et
un piston (4) qui est abrité dans le passage (21) de façon à pouvoir être déplacé dans la direction longitudinale, qui se prolonge en direction de l'extérieur de l'extrémité postérieure ouverte (224, 932) de façon à ce qu'il soit possible de le faire fonctionner manuellement, qui présente une cavité (41) qui se prolonge dans la direction longitudinale et contient un liquide à une pression réduite et qui est couplé à la canule à aiguille (32) à l'intérieur du passage (21) de sorte que la canule à aiguille (32) puisse être rétractée dans la cavité (41) quand la canule à aiguille (32) est soumise à une force de succion produite en résultat d'une différence de pression entre l'air ambiant et la pression réduite, ledit cathéter intraveineux étant **caractérisé par** :
un pavillon de cathéter (5) comprenant
une paroi enveloppant l'extrémité (512) qui entoure un axe et délimite un orifice de passage (516) qui se prolonge le long de l'axe,
une paroi de la gaine (511) qui est à l'opposé de ladite paroi enveloppant l'extrémité (512) le long de l'axe et dont une surface de la paroi interne de la gaine entoure l'axe et délimite un orifice d'insertion (517) plus gros que ledit orifice de passage (516), ladite surface de la paroi interne de la gaine étant adaptée de façon à être arrimée par rapport à la région antérieure enveloppante (2211) de la surface de la paroi externe enveloppante (221) du corps (2) pour produire un alignement entre le passage (21) et ledit orifice de passage (516), et
une paroi tubulaire intermédiaire (51) interposée entre ladite paroi enveloppant l'extrémité (512) et ladite paroi de la gaine (511), qui délimite un conduit (513) mettant en communication ledit orifice d'insertion (517) avec ledit orifice de passage (516) et qui est adaptée de façon à ce que la canule à aiguille (32) passe au travers quand ladite surface de la paroi interne de la gaine est arrimée par rapport à la région antérieure enveloppante (2211) de la surface de la paroi externe enveloppante (221) du corps (2), et présente une embouchure de communication (514) qui la prolonge de communique ledit conduit (513) avec l'air ambiant ; et
un cathéter tubulaire (6) comportant un segment proximal (61) qui est disposé dans ledit orifice de passage (516) et se prolonge le long de l'axe et communique avec ledit conduit (513), et un segment distal (62) qui se prolonge à partir dudit segment proximal (61) le long de l'axe et se projette en direction de l'extérieur de ladite paroi enveloppant l'extrémité (512) et qui est adapté pour être glissé sur la canule à aiguille (32) et permettre une extension de la canule à aiguille (32) en direction de l'extérieur de celle-ci quand la canule à aiguille (32) est amenée à passer au travers dudit conduit (513) ;
ladite paroi tubulaire intermédiaire (51) présentant une surface de paroi tubulaire interne qui délimite ledit conduit (513) et une surface de paroi tubulaire externe à l'opposé de ladite surface de paroi tubulaire interne en directions radiales, ladite surface de paroi tubulaire interne formant un épaulement de retenue (518) avec ladite paroi enveloppant l'extrémité (512) et ledit épaulement de retenue (518) étant en confrontation avec ledit conduit (513) le long de l'axe, ledit segment proximal (61) se prolongeant le long de l'axe et se projetant en direction de l'extérieur dudit orifice de passage (516) et se terminant par une portion à brides (611) en aboutement contre et retenue par ledit épaulement de retenue (518) ;
ladite embouchure de communication (514) se prolongeant radialement au travers desdites surfaces interne et externe de la paroi tubulaire ;
ladite surface de la paroi interne de la gaine délimitant une zone de séparation qui entoure l'axe;
ledit cathéter intraveineux comprenant également :
un élément barrière (54) qui s'accouple à ladite surface de la paroi interne de la gaine au niveau de ladite zone de séparation ; et
un élément rapporté tubulaire (55) présentant une extrémité antérieure rétrécie (554) qui est raccordée au dit élément barrière (54) et délimite un passage le long de l'axe et une extrémité postérieure évasée (552) qui se prolonge à partir de ladite extrémité antérieure rétrécie (554) le long de l'axe, ladite extrémité antérieure rétrécie (552) présentant surface interne aboutée entourant l'axe et délimitant un passage liquidien (551) et une embouchure de réentrée (555) qui se prolonge radialement et communique avec ledit passage liquidien (551) et qui est à l'opposé de ladite embouchure le communication (514) par rapport à ladite zone de séparation, ladite surface interne aboutée de ladite extrémité postérieure évasée (552) étant adaptée pour s'accoupler à ladite région antérieure enveloppante (2211) de ladite surface de la paroi externe (221) enveloppant ledit corps (2) et produisant ainsi un alignement entre ladite voie de passage direct et ledit passage (21) dudit corps (2) afin de permettre l'extension de ladite canule à aiguille (32) au travers de ladite voie de passage direct et en direction de l'extérieur dudit cathéter tubulaire (6).
